# EUROPEAN PATENT APPLICATION

(11) **EP 0 860 503 A1**
(43) Date of publication of application: **26.08.1998**
(21) Application number: 97939226.3
(22) Date of filing: 09.09.1997
(51) Int. Cl.: C12N 15/31, C12Q 1/68

(54) **MATERIAL AND METHOD FOR DETECTING FUNGI**

(30) Priority: 09.09.1996 JP 260310/96
(71) Applicant: SS Pharmaceutical Co., Ltd., Tokyo 103 (JP)
(72) Inventor: YOKOYAMA, Koji, Hanamigawa-ku, Chiba-shi, Chiba 262 (JP); WANG, Li, Chiba-shi, Chiba 260 (JP)
(74) Representative: Nicholls, Kathryn Margaret
(86) International application number: JP9703164
(87) International publication number: WO9810073

(57) **Abstract**

Nucleic acids for detecting fungi, which have sequences shown in SEQ ID NOs. 3, 57 to 75 are disclosed. The nucleic acids for detecting fungi may be used as probes or primers for gene amplification. In case of using them as primers, detection and identification of fungi belonging to the genus *Aspergillus* may be attained because the sizes of the amplified nucleic acid fragments vary depending on the species of the fungi belonging to the genus *Aspergillus* if the primers are appropriately selected.

## Description

### TECHNICAL FIELD

The present invention relates to a nucleic acid for detecting fungi and a method for detecting fungi using the same.

### BACKGROUND ART

Major pathogenic fungi causing deep mycosis include those belonging to the genus *Candida* (hereinafter referred to as "*C.*" for short), to the genus *Aspergillus* hereinafter referred to as 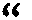 *A.* 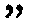 for short) and to the genus "*Cryptococcus*" (hereinafter referred to as "*Cr.*" for short). Major pathogenic fungi belonging to the genus *Candida* include *C. albicans*, *C. kefyr*, *C. glabrata* and *C. tropicalis*, and major pathogenic fungi belonging to the genus *Cryptococcus* include *Cr. neoformans*. Other pathogenic fungi causing deep mycosis, which are as important as those belonging to the genus *Candida*, are those belonging to the genus *Aspergillus*. The species causing deep mycosis, which belong to the genus *Aspergillus* include *A. fumigatus*, *A. flavus*, *A. niger* and niger group, *A. nidulans* and *A. terreus*. It is desired to quickly detect, group and identify these species.

In recent years, deep mycosis in the form of opportunistic infectious disease increases among immunodeficient patients. Since immunodeficient patients often have serious underlying diseases, it is necessary to identify the etiologic fungus at an early stage and to perform a proper therapy. Diagnosis of deep mycosis is based on the blood culture method. However, this method has a drawback in that the detection sensitivity is not satisfactory. Although diagnostics employing antibodies have been commercially available, diagnosis employing antibodies are useless for immunodeficient patients. Under these circumstances, methods for directly detecting antigens and other fungal components were developed recently. For example, methods for diagnosis by detecting mannan, D-arabinitol, glucan or the like has been developed. On the other hand, with the progress of molecular biology, methods for diagnosis comprising extracting DNAs of various pathogenic fungi followed by detection of nucleic acids by using PCR method have been developed. In the field of deep mycosis, a method for diagnosis by detecting ribosomal RNA has been proposed (Clinical Pathology, Vol. 43, Supplemental Edition, p.119, 1995).

### DISCLOSURE OF THE INVENTION

An object of the present invention is to provide a nucleic acid used for detecting pathogenic fungi causing deep mycosis, especially the fungi belonging to the genus *Aspergillus*, and to provide a method for detecting pathogenic fungi causing deep mycosis, especially the fungi belonging to the genus *Aspergillus*, by using the nucleic acid according to the present invention, which method is simple and quick, and has high specificity.

The present inventors intensively studied the genes of the pathogenic fungi causing deep mycosis, especially the fungi belonging to the genus *Aspergillus*, and turned attention to the mitochondrial cytochrome b genes. The present inventors prepared various nucleic acids for amplifying parts of the mitochondrial cytochrome b genes. Among the nucleic acids, the present inventors searched primers by which the fungal cytochrome b gene can be amplified. As a result, using the nucleic acids having the nucleotide sequences shown in SEQ ID NOs. 1-4 in the SEQUENCE LISTING, the present inventors succeeded in amplifying parts of the mitochondrial cytochrome b genes of fungi and in determining the nucleotide sequences of the amplified regions. The present inventors further studied based on the determined nucleotide sequences, to discover sequences which are specific to each of the species of the fungi belonging to the genus *Aspergillus*. Based on the specific sequences, the present inventors designed primers each of which is specific to the respective species. For example, the present inventors provided primers specific to *A. fumigatus*, which have the nucleotide sequences shown in SEQ ID NO. 10, 65, 68 and 69 in the SEQUENCE LISTING; primers specific to *A. flavus* which have the nucleotide sequences shown in SEQ ID NO. 11, 12, 66 and 67; primer specific to *A. niger*, which have the nucleotide sequences shown in SEQ ID NO. 13, 64, 70 and 71; primers specific to *A. nidulans*, which have the nucleotide sequences shown in SEQ ID NO. 14, 63, 72 and 73; and primers specific to *A. terreus*, which have the nucleotide sequences shown in SEQ ID NO. 15, 62, 74 and 75.

Based on these, the present inventors further prepared nucleic acids for detecting, grouping or identifying the fungi belonging to the genus *Aspergillus* simply, quickly, specifically and with high sensitivity, and established a method for detection using the nucleic acids, thereby completing the present invention. The present invention further provides a primer having the nucleotide sequence shown in SEQ ID NO. 57, which is an improvement of the primers having the nucleotide sequences shown in SEQ ID Nos. 1 and 2. By this primer, *Cunninghamella bertholletiae* and *Mucor circinelloides* can be detected in addition to the above-mentioned fungi.

That is, the present invention provides a primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 57 (provided that one or more thymine at optional site(s) may be substituted with uracil) or in the nucleotide sequence complementary thereto. The present invention also provides a primers for amplifying a fragment of mitochondrial cytochrome b gene of fungi, consisting essentially of a nucleic acid fragment of which nucleotide sequences are sequences of not less than 10 consecutive nucleotides in the nucleotide sequence shown in SEQ ID NO. 3 or 58-61 (provided that one or more thymine at optional site(s) may be substituted with uracil) or in the nucleotide sequence complementary thereto. The present invention also provides nucleic acids for detecting *Aspergillus fumigatus* (SEQ ID NO. 5), *Aspergillus flavus* (SEQ ID NO. 6), *Aspergillus niger* (SEQ ID NO. 7), *Aspergillus nidulans* (SEQ ID NO. 8) or *Aspergillus terreus* (SEQ ID NO. 9), each of which consists essentially of a nucleic acid fragment of which nucleotide sequence is 10 to 100 consecutive nucleotides in the nucleotide sequence shown in SEQ ID NO. 5, 6, 7, 8 or 9 (provided that one or more thymine at optional site(s) may be substituted with uracil) or in the nucleotide sequence complementary thereto. The present invention further provides nucleic acids for detection of *Aspergillus fumigatus*, each of which consists essentially of a nucleic acid fragment of which nucleotide sequence is a sequence of not less than 10 consecutive nucleotides in the nucleotide sequence shown in SEQ ID NO. 5, the 3'-end of said nucleic acid being the 24nt "T", 99nt 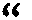 T 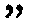 , 144nt 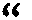 T 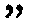 , 252nt 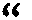 C 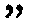 , 277nt 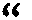 G 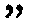 , 304nt 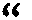 G 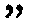 , 312nt 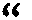 A 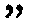 or 393nt 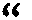 G 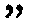 in the nucleotide sequence shown in SEQ ID NO. 5, or each of which consists essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with any of said 3 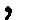 -ends of said nucleic acids. The present invention further provides a nucleic acid for detection of *Aspergillus flavus*, which consists essentially of a nucleic acid fragment of which nucleotide sequence is a sequence of not less than 10 consecutive nucleotides in the nucleotide sequence shown in SEQ ID NO. 6, the 3'-end of the nucleic acid being the 174nt "Y" in the nucleotide sequence shown in SEQ ID NO. 6, or each of which consists essentially of a nucleic acid fragment of which nucleotide sequence is a sequence of not less than 10 consecutive nucleotides in the nucleotide sequence complementary to the sequence shown in SEQ ID NO. 6, of which 3'-end is the base that pairs with any of said 3 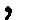 -ends of said nucleic acids. The present invention further provides nucleic acids for detection of *Aspergillus niger*, each of which consists essentially of a nucleic acid fragment of which nucleotide sequence is a sequence of not less than 10 consecutive nucleotides in the nucleotide sequence shown in SEQ ID NO. 7, the 3'-end of the nucleic acid being the 42nt "A", 69nt 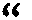 C 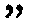 , 126nt 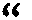 C 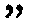 , 207nt 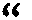 A 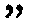 , 213nt 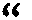 A 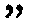 , 246nt 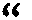 A 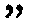 or 396nt 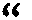 G 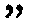 in the nucleotide sequence shown in SEQ ID NO. 7, or each of which consists essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with any of said 3 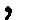 -ends of said nucleic acids. The present invention further provides nucleic acids for detection of *Aspergillus nidulans*, each of which consists essentially of a nucleic acid fragment of which nucleotide sequence is a sequence of not less than 10 consecutive nucleotides in the nucleotide sequence shown in SEQ ID NO. 8, the 3'-end of the nucleic acid being the 60nt "T", 221nt 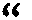 C 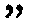 , 271nt 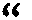 A 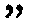 , 285nt 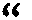 A 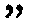 , 366nt 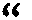 T 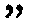 or 387nt 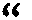 A 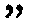 in the nucleotide sequence shown in SEQ ID NO. 8, or each of which consists essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with any of said 3 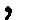 -ends of said nucleic acids. The present invention further provides nucleic acids for detection of *Aspergillus terreus*, each of which consists essentially of a nucleic acid fragment of which nucleotide sequence is a sequence of not less than 10 consecutive nucleotides in the nucleotide sequence shown in SEQ ID NO. 9, the 3'-end of the nucleic acid being the 25nt "T", 48nt 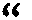 T 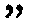 , 162nt 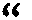 T 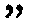 , 225nt 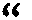 C 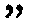 , 261nt 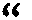 C 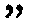 , 339nt 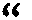 C 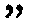 , 360nt 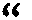 A 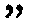 , 375nt 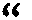 A 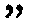 or 411nt 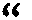 A 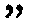 in the nucleotide sequence shown in SEQ ID NO. 9, or each of which consists essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with any of said 3 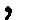 -ends of said nucleic acids. The present invention further provides a method for detecting and/or identifying fungi belonging to the genus *Aspergillus* comprising amplifying a fragment of mitochondrial cytochrome b gene of fungi belonging to the genus *Aspergillus* by a gene amplification method using at least one forward primer selected from the group consisting of nucleic acid fragments of which nucleotide sequences are sequences of not less than 10 consecutive nucleotides in the nucleotide sequences shown in SEQ ID NOs. 1, 2 and 57 (provided that one or more thymine at optional site(s) may be substituted with uracil), and at least one reverse primer selected from the group consisting of nucleic acid fragments of which nucleotide sequences are sequences of not less than 10 consecutive nucleotides in the nucleotide sequences complementary to the sequences shown in SEQ ID NOs. 11-15, and 62-65 (provided that one or more thymine at optional site(s) may be substituted with uracil). The present invention further provides a method for detecting and/or identifying fungi belonging to the genus *Aspergillus* comprising amplifying a fragment of mitochondrial cytochrome b gene of fungi belonging to the genus *Aspergillus* by a gene amplification method using at least one forward primer selected from the group consisting of nucleic acid fragments of which nucleotide sequences are sequences of not less than 10 consecutive nucleotides in the nucleotide sequences shown in SEQ ID NOs 66 to 75 (provided that one or more thymine at optional site(s) may be substituted with uracil), and at least one reverse primer selected from the group consisting of nucleic acid fragments of which nucleotide sequences are sequences of not less than 10 consecutive nucleotides in the nucleotide sequences complementary to the sequences shown in SEQ ID NOs. 3, 4, and 58 to 61 (provided that one or more thymine at optional site(s) may be substituted with uracil). The present invention still further provides a method for detecting and/or identifying fungi belonging to the genus *Aspergillus* comprising amplifying a fragment of mitochondrial cytochrome b gene of fungi belonging to the genus *Aspergillus* by a gene amplification method using at least one forward primer selected from the group consisting of nucleic acid fragments of which nucleotide sequences are sequences of not less than 10 consecutive nucleotides in the nucleotide sequences shown in SEQ ID NOs. 1, 2 and 57 (provided that one or more thymine at optional site(s) may be substituted with uracil), and at least one reverse primer selected from the group consisting of nucleic acid fragments of which nucleotide sequences are sequences of not less than 10 consecutive nucleotides in the nucleotide sequences complementary to the sequences shown in SEQ ID NOs. 3, 4, and 58 to 61 (provided that one or more thymine at optional site(s) may be substituted with uracil).

By the present invention, detection, grouping and identification of the pathogenic fungi, especially those belonging to the genus *Aspergillus*, can be made quickly, simply, specifically and with high sensitivity, of which grouping and identification hitherto have been time-consuming. The nucleic acids according to the present invention may be used either as primers or as probes for detection. Further, the products amplified by using the primers may be detected with the probes or restriction enzymes, thereby the sensitivity and specificity are further promoted. Thus, the significance of the present invention in the clinical field is large.

### BRIEF DESCRIPTION OF THE DRAWING

Fig. 1 is a schematic view showing the electrophoresis pattern obtained by amplifying mitochondrial cytochrome b genes of various fungi by PCR using various primers which are examples according to the present invention, and electrophoresing the amplified product.

Fig. 2 is a schematic view showing the electrophoresis pattern obtained by amplifying mitochondrial cytochrome b genes of various fungi by PCR using various primers which are other examples according to the present invention, and electrophoresing the amplified product.

Fig. 3 is a schematic view showing the electrophoresis pattern obtained by amplifying mitochondrial cytochrome b genes of various fungi by PCR using various primers which are still other examples according to the present invention, and electrophoresing the amplified product.

Fig. 4 is a schematic view showing the electrophoresis pattern obtained by amplifying mitochondrial cytochrome b genes of various fungi by PCR using various primers which are still other examples according to the present invention, and electrophoresing the amplified product.

### BEST MODE FOR CARRYING OUT THE INVENTION

Mitochondrial cytochrome b gene fragment of fungi may be amplified by hybridizing and elongating the chain of a pair of nucleic acids, one of which being selected from the group consisting of the nucleic acids having the nucleotide sequences shown in SEQ ID NOs. 1, 2 and 57, and another one of which being selected from the group consisting of the nucleic acids complementary to the nucleic acids having the nucleotide sequences shown in SEQ ID NOs. 3, 4 and 58 to 61, with the mitochondrial cytochrome b gene of fungi such as those belonging to the genus *Aspergillus* or *Candida;* and repeating the cycle including the extension reaction of the primers. Among the above-mentioned sequences, those shown in SEQ ID NOs. 1, 2 and 4 were designed based on the sequence of the mitochondrial cytochrome b gene of *A. nidulans* (Waring, R.B. et al., Cell 27, 4-11 (1981)) . On the other hand, there are a plurality of nucleic acids having the nucleotide sequence shown in SEQ ID NO. 57, 3 or 58-61 (the nucleotide sequences shown in SEQ ID NOs. 57, 3, 58-61 are represented by general formulae, respectively). Although each of the nucleic acids having the sequence shown in one of these SEQ ID NOs. may be used individually, it is preferred to use a mixture of the nucleic acids shown by each general formula. By using the mixture, it was attained for the first time to amplify the mitochondrial cytochrome b gene of fungi other than those belonging to *Aspergillus* (excluding *A. terreus*), for example, *A. terreus*, those belonging to the genus *Candida*, *Trichophyton*, *Cunninghamella* or *Mucor*. The nucleic acids consisting essentially of a part of the sequence shown in SEQ ID NO. 57, 3 or 58-61, which part is not less than 10 consecutive nucleotides in the respective sequences may be employed as primers. However, it is preferred to employ primers having the full length of the sequences shown in these SEQ ID Nos., respectively.

Using the above-described various types of nucleic acids, fragments of mitochondrial cytochrome b genes of a number of strains of various fungi were amplified and nucleotide sequences thereof were determined. The sequences each of which is consentaneous to each species are shown in SEQ ID NOs. 5 - 9. The sequences shown in SEQ ID NOs. 5, 6, 7, 8 and 9 are the sequences of the amplified DNA fragments originated from *A. fumigatus*, *A. flavus*, *A. niger*, *A. nidulans* and *A. terreus*, respectively. Using these amplified fragments as templates, and using primers specific to each fungal species, which primers are hereinbelow described, amplification of nucleic acids may be carried out. Alternatively, by digesting the amplified product with one or more restriction enzymes and comparing the pattern of the generated fragments with standard patterns, the product formed by extension of primers may be detected (PCR-RFLP).

The nucleic acids consisting essentially of the nucleotide sequences which are optional 10 to 100 consecutive nucleotides, preferably 15 to 30 consecutive nucleotides, in the nucleotide sequences mentioned above may be used as nucleic acids for detection of the above-mentioned fungal species, respectively. The nucleic acids for detection include primers for amplifying nucleic acids and probes. In cases where the nucleic acids are used as probes, those labeled by a well-known method are used.

As concretely described in the examples below, the present inventors amplified the genes of a number of strains of the fungi belonging to the genus *Aspergillus*, determined the sequences of the amplified genes and carefully compared the sequences, to discover sites each of which is specific to each species. Examples of the specific sites (1 base) include the 24nt 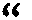 T 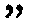 , 99nt 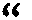 T 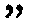 , 144nt 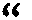 T 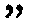 , 252nt 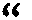 C 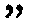 , 277nt 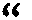 G 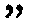 , 304nt "G", 312nt 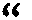 A 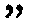 and the 393nt "G" in the sequence shown in SEQ ID NO. 5 for *A*. *fumigatus;* the 174nt "Y" in the sequence shown in SEQ ID NO. 6 for *A. flavus;* the 69nt "C", the 126nt "C", the 207nt "A", 213nt 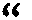 A 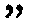 , 246nt 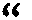 A 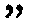 and 396nt 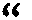 G 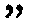 in the sequence shown in SEQ ID NO. 7 for *A. niger;* the 60nt 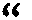 T 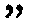 , 221nt "C", 271nt 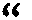 A 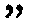 , 285nt 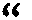 A 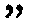 , 366nt 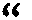 T 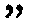 and 387nt 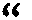 A 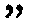 in the sequence shown in SEQ ID NO. 8 for *A. nidulans;* and the 25nt 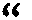 T 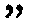 , 48nt 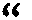 T 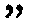 , 162nt 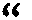 T 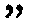 , 225nt 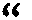 C 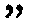 , 261nt 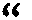 C 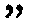 , 339nt 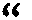 C 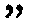 , 360nt 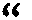 A 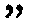 , 375nt "A" and 411nt 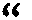 A 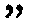 in the sequence shown in SEQ ID NO. 9 for *A. terreus*. These specific sites also exist in the complementary sequences of the sequences shown in SEQ ID NOs. 5 - 9, the bases of these specific sites in the complementary sequences being complementary to the bases mentioned above. By using an oligonucleotide whose 3'-end is the above-mentioned specific site (1 base) as a primer for amplifying the nucleic acid, the mitochondrial cytochrome b gene fragment of the corresponding species alone is amplified. Therefore, each species may be specifically detected, in other words, identification of the species may be carried out.

Examples of the sequences of the nucleic acids (used as reverse primers) which may be employed as primers in combination with the nucleic acid having the sequence shown in SEQ ID NO. 1, 2 and/or 57 (used as forward primers) are shown in SEQ ID NOs. 10 - 15 and 62 - 65. The 3'-end of each of these nucleic acids is one of the specific sites mentioned above in the sequences of SEQ ID NOs 5-9, and these nucleic acids have not less than 10 bases. That is, the nucleic acids having the sequence shown in SEQ ID NOs. 10 and 65, respectively (both of these sequences are complementary chains of SEQ ID NO. 5, of which 3 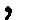 -ends are the 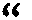 C 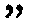 which pairs with the 304nt 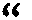 G 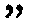 of SEQ ID NO. 5), are for detecting *Aspergillus fumigatus*. The nucleic acids having the sequence shown in SEQ ID NOs. 11 and 12, respectively (both of these sequences are complementary chains of SEQ ID NO. 6, of which 3 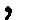 -ends are the 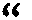 G 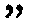 and 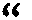 A 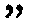 , respectively, which pair with the 174nt 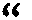 Y 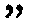 (C or T) of SEQ ID NO. 6), are for detecting *Aspergillus flavus*. The nucleic acids having the sequence shown in SEQ ID NO. 13 (complementary chain of SEQ ID NO. 7, of which 3 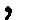 -end is the 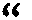 G 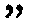 which pairs with the 126nt 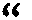 C 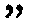 of SEQ ID NO. 7) and SEQ ID NO. 64 (complementary chain of SEQ ID NO. 7, of which 3 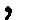 -end is the 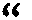 T 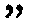 which pairs with the 207nt 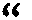 A 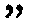 of SEQ ID NO. 7) are for detecting *Aspergillus niger*. The nucleic acids having the sequence shown in SEQ ID NO. 14 (complementary chain of SEQ ID NO. 8, of which 3 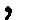 -end is the 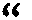 G 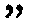 which pairs with the 221nt 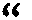 C 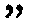 of SEQ ID NO. 8) and SEQ ID NO. 63 (complementary chain of SEC ID NO. 8, of which 3 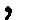 -end is the 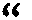 T 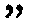 which pairs with the 271nt 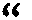 A 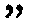 of SEQ ID NO. 8) are for detecting *Aspergillus nidulans*. The nucleic acids having the sequence shown in SEQ ID NO. 15 (complementary chain of SEQ ID NO. 9, of which 3 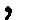 -end is the 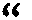 T 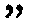 which pairs with the 375nt 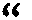 A 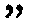 of SEQ ID NO. 9) and SEQ ID NO. 62 (complementary chain of SEQ ID NO. 9, of which 3 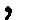 -end is the 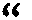 T 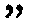 which pairs with the 360nt 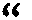 A 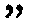 of SEQ ID NO. 9) are for detecting *Aspergillus terreus*.

On the other hand, primers for amplifying nucleic acids, by which identification of species can be carried out, may be designed by combining nucleic acids having the sequences shown in SEQ ID NO. 3, 4, 58, 59, 60 and/or 61 (used as reverse primers) and one of the above-mentioned nucleic acids, whose 3'-end is one of the above-mentioned specific sites (1 base). For example, the nucleic acid for detecting *A. fumigatus* is selected from the nucleic acids whose 3'-ends are any of the 24nt "T", 99nt 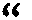 T 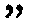 , 144nt 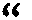 T 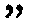 , 252nt 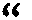 C 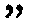 , 277nt 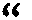 G 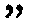 , 304nt 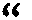 G 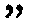 , 312nt 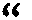 A 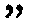 and 393nt 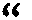 G 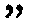 in the sequence shown in SEQ ID NO. 5, which have not less than 10 bases. The nucleic acid for detecting *A. flavus* is selected from the nucleic acids whose 3'-ends are the 174nt "Y" in the sequence shown in SEQ ID NO. 6, which have not less than 10 bases. The nucleic acid for detecting *A. niger* is selected from the nucleic acids whose 3'-ends are any of the 42nt 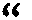 A 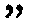 , 69nt "C", 126nt "C", 207nt "A", 213nt 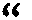 A 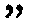 , 246nt 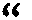 A 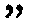 and 396nt 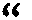 G 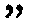 in the sequence shown in SEQ ID NO. 7, which have not less than 10 bases. The nucleic acid for detecting *A. nidulans* is selected from the nucleic acids whose 3'-ends are any of the 60nt 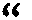 T 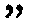 , 221nt "C", 271nt 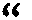 A 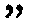 , 285nt 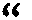 A 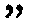 , 366nt 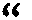 T 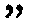 and 387nt 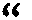 A 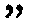 in the sequence shown in SEQ ID NO. 8, which have not less than 10 bases. The nucleic acid for detecting *A. terreus* is selected from the nucleic acids whose 3'-ends are any of the 25nt 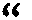 T 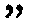 , 48nt 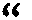 T 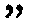 , 162nt 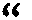 T 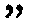 , 225nt 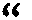 C 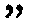 , 261nt 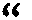 C 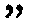 , 339nt 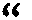 C 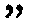 , 360nt 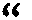 A 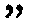 , 375nt 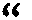 A 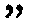 and 411nt 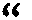 A 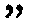 in the sequence shown in SEQ ID NO. 9, which have not less than 10 bases. Preferred examples of these primers which are used as forward primers are shown in SEQ ID NOs. 66-75. The nucleic acids having the sequences shown in SEQ ID NOs. 66 and 67 (both of which 3 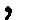 -ends are the 174nt 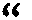 Y 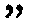 (C or T) of SEQ ID NO. 6) are for detecting *Aspergillus flavus*. The nucleic acids having the sequences shown in SEQ ID NO. 68 (of which 3 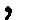 -end is the 304nt 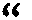 G 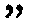 of SEQ ID NO. 5) and SEQ ID NO. 69 (of which 3 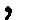 -end is the 312nt 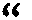 A 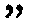 of SEQ ID NO. 5) are for detecting *Aspergillus fumigatus*. The nucleic acids having the sequences shown in SEQ ID NO. 70 (of which 3 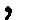 -end is the 126nt 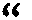 C 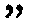 of SEQ ID NO. 7) and SEQ ID NO. 71 (of which 3 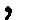 -end is the 213nt 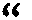 A 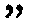 of SEQ ID NO. 7) are for detecting *Aspergillus niger*. The nucleic acids having the sequences shown in SEQ ID NOs. 72 (of which 3 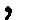 -end is the 221nt 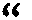 C 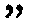 of SEQ ID NO. 8) and SEQ ID NO. 73 (of which 3 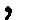 -end is the 285nt 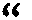 A 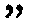 of SEQ ID NO. 8) are for detecting *Aspergillus nidulans*. The nucleic acids having the sequences shown in SEQ ID NOs. 74 and 75 (both of which 3 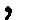 -ends are the 375nt 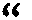 A 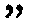 of SEQ ID NO. 9) are for detecting *Aspergillus fumigatus*.

These oligonucleotides may be used not only as primers, but also as probes specific to each species after attaching an appropriate label thereto. The above-mentioned nucleic acids may be used as primers or probes as long as they have consecutive not less than 10 bases starting from the above-mentioned 3'-ends. The number of bases of the nucleotides is preferably 10 to 100, more preferably 15 to 30. As for the nucleic acids having the parts of the sequences shown in SEQ ID NOs. 10 - 15 and 62-75, it is preferred that they have the full length of these sequences.

In the nucleotide sequences of the nucleic acids according to the present invention, which are shown in the SEQUENCE LISTING, thymine (T) may be uracil (U). That is, the nucleic acids according to the present invention include both DNAs and RNAs. Further, the nucleic acid according to the present invention may be a DNA which contains uridine residue "U" in place of "T" at optional site(s), and may be an RNA which contains thymine residue "T" in place of "U" at optional site(s). Further, complementary chains of the nucleic acids according to the present invention are also within the scope of the present invention. This is because that the DNAs of fungi are double-stranded, so that the complementary chain of the nucleic acid according to the present invention can also hybridize with the complementary chain of the nucleic acid with which the nucleic acid according to the present invention hybridizes. Still further, as long as the nucleic acid can be applied to the above-described uses, the nucleic acids having one or more point mutations such as deletion, insertion and substitution, or those having modified nucleotide(s) are also within the scope of the present invention.

The method for obtaining the nucleic acids according to the present invention is not restricted. Preferably, they are chemically synthesized. By this method, the nucleic acids with uniform quality may be obtained easily and inexpensively in large scale. Such chemical synthesis may be carried out by a conventional method such as the phosphite method (phosphoamidite method) or the phosphotriester method (phosphoric acid triester method).

The method for detecting, grouping or identifying a fungus belonging to the genus *Aspergillus* according to the present invention is characterized by using the above-described nucleic acid for detecting, grouping or identifying a fungus belonging to the genus *Aspergillus*. The method includes (1) the method in which the nucleic acid is used as a probe, so as to detect, group or identify a fungus belonging to the genus *Aspergillus*, and (2) the method in which the nucleic acid is used as a primer for PCR or the like and the amplified product is measured, so as to detect, group or identify a fungus belonging to the genus *Aspergillus*. The nucleic acid used in these methods may be a labeled nucleic acid. Preferably, the nucleic acid is a labeled nucleic acid because the fungi belonging to the genus *Aspergillus* may be detected more easily. Examples of the label include antigens, haptens, enzymes, fluorescent substances, luminescent substances, enzyme substrates, radioactive substances and insoluble carriers. The label may be attached to either the end of the nucleic acid or to an inner portion of the sequence. The label may be attached to any of the saccharide, phosphate and base moieties.

The method for detecting, grouping or identifying fungi belonging to the genus *Aspergillus* using the nucleic acid according to the present invention as probes or primers include, for example, the following methods (1) and (2).
(1) In this method, the nucleic acid for detecting, grouping or identifying fungi belonging to the genus *Aspergillus* is used as a probe. That is, the nucleic acid is hybridized with the target nucleic acid in a sample and then the probe is detected, thereby detecting the target nucleic acid. In this method, it is preferred to use the nucleic acid for detecting, grouping or identifying fungi belonging to the genus *Aspergillus*, which is labeled with the above-mentioned label. This method may be carried out by hybridizing the labeled nucleic acid as a probe with the gene of a fungus belonging to the genus *Aspergillus* in a sample, and then detecting the hybrid between the probe and the gene of *Aspergillus*, or the non-hybridized probe by an appropriate method for detecting the label in the probe.
   The hybridization between the gene of *Aspergillus* in the sample and the probe may be carried out by pretreating the sample to purify nucleic acids, mixing the resultant with the labeled nucleic acid as a probe, and incubating the mixture at a temperature between room temperature and 70°C for 10 minutes to 48 hours. The target nucleic acid may be amplified by using the nucleic acid according to the present invention as a primer.
(2) In this method, using the nucleic acid for detecting fungi belonging to the genus *Aspergillus* according to the present invention as a primer, extension reaction of the primer is carried out using a DNA polymerase or the like, thereby specifically amplifying the cytochrome b gene fragment of a fungus belonging to the genus *Aspergillus* alone, and detecting the amplified product, thereby detecting, grouping or identifying *Aspergillus*. In this method, as the primer, the nucleic acid according to the present invention labeled with the above-mentioned label may also be used. An example of the gene amplification method is the PCR method. However, the gene amplification method is not restricted to the PCR method, but any gene amplification method in which a short oligonucleotide is used as a primer for initiation of the gene synthesis may be employed. The method for detecting *Aspergillus* according to the present invention may be carried out by detecting the amplified product, that is, the cytochrome b gene fragment. Alternatively, detection of *Aspergillus* may also be carried out by digesting the amplified cytochrome b gene fragment with one or more restriction enzymes and comparing the pattern of the sizes of the generated fragments with standard patterns. In this case, one restriction enzyme may be used or a plurality of restriction enzymes may be used in combination. The method for detecting the amplified cytochrome b gene fragment or the gene fragments generated by digesting the amplified product with one or more restriction enzymes is not restricted, and ordinary methods for detecting genes (e.g., electrophoresis) may be employed. The amplified product may be, for example, separated by electrophoresis, and the amplified gene may easily be detected as a band which is clear enough to be specifically detected. In cases where a plurality of primers are used in order to detecting, grouping or identifying a plurality of pathogenic fungi, the primers may be designed such that bands corresponding to different molecular weights are detected for the respective pathogenic fungi, thereby enabling the grouping or identification of each pathogenic fungus. By using deoxyribonucleotides (dATP, dTTP, dGTP, dCTP) or by using ribonucleotides (ATP, UTP, GTP, CTP) which are labeled with an appropriate label as the materials for the amplification reaction, the amplified product may be directly detected with high sensitivity. Alternatively, by using a labeled nucleic acid obtained by labeling the nucleic acid for detecting, grouping or identifying *Aspergillus* according to the present invention, the amplified product may be directly detected with high sensitivity. In these cases, preferred examples of the labels include radioactive substances, fluorescent substances, luminescent substances and luminescence-inducing substances.

An example of the method for detecting, grouping or identifying *Aspergillus* using the nucleic acid according to the present invention will now be described in more detail. Nucleic acids are extracted from the sample and nucleic acid-extension reaction such as PCR is carried out using the nucleic acid having the sequence shown in SEQ ID NO. 2 and a mixture of the nucleic acids having the sequences shown in SEQ ID NOs. 10 - 15 as primers. In this method, if *A. fumigatus* exists in the sample, a nucleic acid having a length of 323 bp is amplified; if *A.* *flavus* exists, nucleic acid having a length of 193 bp or 194 bp is amplified; if *A. niger* exists, a nucleic acid having a length of 148 bp is amplified; if *A. nidulans* exists, a nucleic acid haying a length of 244 bp is amplified; and if *A. terreus* exists, a nucleic acid having a length of 397 bp is amplified, respectively. By separating and detecting these nucleic acids having different molecular weights by electrophoresis or the like, what species of *Aspergillus* exists in the sample can be determined. In cases where nucleic acid-extension reaction such as PCR is carried out using the nucleic acid having the sequence shown in SEQ ID NO. 2 and a mixture of the nucleic acids having the sequences shown in SEQ ID NOs. 62 - 65 as primers, if *A. fumigatus* exists in the sample, a nucleic acid having a length of 329 bp is amplified; if *A. flavus* exists, nucleic acid having a length of 193 bp or 194 bp is amplified; if *A. niger* exists, a nucleic acid having a length of 231 bp is amplified; if *A. nidulans* exists, a nucleic acid having a length of 294 bp is amplified; and if *A. terreus* exists, a nucleic acid having a length of 379 bp is amplified, respectively. In cases where nucleic acid-extension reaction such as PCR is carried out using the nucleic acid having the sequence shown in SEQ ID NO. 57 (mixture) and a mixture of the nucleic acids having the sequences shown in SEQ ID NOs. 62 - 65 as primers, if *A. fumigatus* exists in the sample, a nucleic acid having a length of 330 bp is amplified; if *A. flavus* exists, nucleic acid having a length of 194 bp or 195 bp is amplified; if *A. niger* exists, a nucleic acid having a length of 232 bp is amplified; if *A. nidulans* exists, a nucleic acid having a length of 295 bp is amplified; and if *A. terreus* exists, a nucleic acid having a length of 380 bp is amplified, respectively. In cases where nucleic acid-extension reaction such as PCR is carried out using the nucleic acidS having the sequences shown in SEQ ID NO. 3, 58, 59, 60 and/or 61 (each sequence is a mixture) and a mixture of the nucleic acids having the sequences shown in SEQ ID NOs. 66 - 75 as primers, if *A. fumigatus* exists in the sample, nucleic acidS having lengths of 163 bp (by SEQ ID NO. 68) and 146 bp (by SEQ ID NO. 69) are amplified; if *A. flavus* exists, nucleic acid having a length of 287 bp is amplified; if *A. niger* exists, nucleic acids having lengths of 337 bp (by SEQ ID NO. 70) and 245 bp (by SEQ ID NO. 71) are amplified; if *A. nidulans* exists, nucleic acids having lengths of 236 bp (by SEQ ID NO. 72) and 180 bp (by SEQ ID NO. 73) are amplified; and if *A. terreus* exists, nucleic acids having lengths of 86 bp (by SEQ ID NO. 74) and 83 bp (by SEQ ID NO. 75) are amplified, respectively. The nucleic acids having sequences shown in SEQ ID NOs. 66-75 include two primers per each *Aspergillus* species. Therefore, by selecting one primer per each *Aspergillus* species, and using the selected totally 5 primers as a mixture, the above-mentioned 5 *Aspergillus* species may be simultaneously detected and identified.

The reagent kit for detecting *Aspergillus*, which utilizes the present invention, is characterized by including the nucleic acid for detecting *Aspergillus* according to the present invention or a labeled nucleic acid obtained by labeling the nucleic acid according to the present invention. As long as the kit includes the above-mentioned nucleotide, the kit is within the scope of the present invention, and the kit may further comprise other components such as a reagent for detecting a label, and a buffer solution. The nucleic acid in the reagent kit according to the present invention may be used as either a primer or a probe. The method in which the nucleic acid as a primer and the method in which the nucleic acid as a probe are different only in the means for detecting the target nucleic acid, and the essential features of these methods about detection of *Aspergillus* are the same. In cases where the nucleic acid is used as a probe, the reagent kit may be used for detection of *Aspergillus* by the above-described method (1), and in cases where the nucleic acid is used as a primer, the reagent kit may be used for detection of *Aspergillus* by the above-described method (2).

In cases where the nucleic acid in the reagent kit is used as a probe, the reagent kit according to the present invention may include a reagent for detecting the label and a buffer solution, in addition to the nucleic acid or labeled nucleic acid for detecting *Aspergillus*. In cases where the nucleic acid is used as a primer, the reagent kit according to the present invention may include a nucleic acid-synthesizing enzyme (e.g., DNA polymerase, RNA polymerase, reverse transcriptase and the like), deoxyribonucleotides (dATP, dTTP, dGTP and dCTP), ribonucleotides (ATP, UTP, GTP and CTP), one or more restriction enzymes, buffer solution and the like. The nucleic acid according to the present invention may be bound to a solid carrier and used as a capturing probe. In this case, using the capturing probe and a labeled probe in combination, sandwich assay may be carried out. Alternatively, the target nucleic acid may be labeled and captured. Further, the nucleic acid may be labeled with biotin, and after hybridization, the nucleic acid may be captured by an avidin-bound carrier. In the sandwich assay, if the nucleic acid according to the present invention is used as one of the probes, specific measurement may be obtained by virtue of the nucleic acid according to the present invention, so that there is no problem even if the specificity of the other nucleic acid is a little bit low.

The present invention will now be described by way of examples thereof. It should be noted that the present invention is not restricted to the examples described below.

### Example 1 (Synthesis of Various DNAs)

Synthesis of DNAs having the sequences shown in SEQ ID NOs. 1 to 4, 10 to 15 and 57 to 75 in the SEQUENCE LISTING were entrusted to a commercial DNA synthesis service. The DNAs having the sequences shown in SEQ ID NOs. 1 to 4, 10 to 15 and 57 to 75 will be referred to as Nucleic Acid 1 to 4, 10 to 15 and 57 to 75, respectively.

### Example 2 (Amplification Reaction of Cytochrome b Genes of A. fumigatus)

Cells of strains of *A. fumigatus* (*A. fumigatus* IFM 40804, 40806, 40807, 40819, 41206, 41392, 45916, 45917, 46980, 5355) were treated with 75% ethanol to kill the cells. After collecting the cells of each of the strains by centrifugation at 1500 x g for 10 minutes, about 40 ml of extraction buffer (0.9 M sorbitol, 10 mM EDTA, 10 mM Tris-HCl buffer, pH7.1) was added to the cells of each strain and the resultant was well mixed. The mixture was then centrifuged at 1500 x g for 10 minutes and the supernatant was discarded. Then 30 ml of the same buffer was added and the supernatant generated by the centrifugation as mentioned above was discarded, followed by addition of 9 ml of the same buffer. To the resultant, 1 ml of solution of Zymolyase (commercially available from SEIKAGAKU CORPORATION) (10 mg/ml) in the same buffer was added and the resulting mixture was incubated at 37°C for 1 hour. Glass beads (0.9 - 1.3 mm) were added in the precipitation of cells, and the cells were disrupted by a vortex mixer for 1 - 2 minutes. The resultant was centrifuged at 1500 x g for 10 minutes and the recovered supernatant was centrifuged at 20,000 x g for 15 minutes to obtain a precipitate. The precipitate was washed with the extraction buffer and centrifuged again at 20,000 x g for 15 minutes, to obtain mitochondrial fragment of each strain of *A. fumigatus*. To each of the thus obtained mitochondrial fragments, 0.5 ml of 1 mg/ml of protease K was added and the resultant was incubated at 37°C for 1 hour. To this, 1 ml of a mixture of phenol/chloroform/isoamyl alcohol (25:24:1) was added and the mixture was well agitated by shaking, followed by centrifugation at 1500 x g for 10 minutes to recover the supernatant. To the supernatant, 1 ml of phenol saturated with water was added and the mixture was well agitated by shaking, followed by centrifugation at 1500 x g for 10 minutes to recover the supernatant. To the obtained supernatant, 0.1 ml of 3 M sodium acetate/0.1 M magnesium chloride solution was added and 3 ml of ethanol was added, followed by leaving the resultant to stand at -20°C overnight. The resultant was centrifuged at 20,000 x g for 10 minutes to recover a precipitate and this precipitate was washed with 75% ethanol at -20°C, followed by centrifugation at 1500 x g for 10 minutes. The supernatant was discarded to obtain nucleic acids. The obtained nucleic acids were dried and stored at -20°C.

Using these DNAs as templates, and using the pair of DNA 1 (SEQ ID NO. 1) and DNA 3 (SEQ ID NO. 3; for IFM40804, 40819) or the pair of DNA 1 and DNA 4 (SEQ ID NO. 4; for IFM40806, 40807, 41206, 41392, 45916, 45917, 46980, 5355) as primers, PCR was performed under the conditions described below, to amplify a part of each cytochrome b gene. The PCR was performed using TaKaRa PCR Amplification Kit (R011) commercially available from TAKARA SHUZO CO., LTD. and a DNA amplifier (MIR-D30) commercially available from SANYO.

The composition of the reaction mixture was as follows:

| | |
|---|---|
| x10 PCR buffer (contained in kit) | 5 µl |
| PCR dNTP mixture solution (contained in kit) | 4 µl |
| *Taq* DNA polymerase (contained in kit) | 1 µl (2.5 U) |
| DNA 1 | 1 µl |
| DNA 3 or 4 | 1 µl |
| Mitochondrial DNA | 1 µl |
| Water | balance to total volume of 50 µl |

The reaction conditions were as follows:
Denaturing: 94°C, 1 minute
Annealing: 50°C, 1 minute
Extension: 72°C, 2 minutes

Each of the amplified DNAs was separated and purified by agarose gel electrophoresis and the nucleotide sequence thereof was determined by the following method.

That is, using a DNA sequencer (ABI Prism 377) commercially available from PERKIN ELMER, the sequence was determined by the dye terminator method according to the instructions by the manufacturer of the DNA sequencer. DNA 2 (SEQ ID NO. 2) was used as the forward primer for sequencing, and DNA 3 (for IFM40804, 40819) or DNA 4 (for IFM40806, 40807, 41206, 41392, 45916, 45917, 46980, 5355) was used as the reverse primer for sequencing. The determined nucleotide sequences are shown in SEQ ID NOs. 16 to 25 in the SEQUENCE LISTING.

### Example 3 (Amplification Reaction of Cytochrome b Genes of A. flavus)

By the same method as in Example 2, parts of the cytochrome b genes of strains of *A. flavus* (*A. flavus* IFM40603, 40800, 41087, 41933, 45909, 45910, 45911, 46870, 5364, 5366) were amplified by PCR and sequences thereof were determined. As the primer pair, the pair of DNA 2 and DNA 3 (for IFM41933, 45910, 5366) or the pair of DNA 2 and DNA 4 (for IFM40603, 40800, 41087, 45909, 45911, 46870, 5364) was used. In sequencing, DNA 2 was used as the forward primer and DNA 3 (for IFM41933, 45910, 5366) or DNA 4 (for IFM40603, 40800, 41087, 45909, 45911, 46870, 5364) was used as the reverse primer. The determined nucleotide sequences are shown in SEQ ID NOs. 26 to 35 in the SEQUENCE LISTING.

### Example 4 (Amplification Reaction of Cytochrome b Genes of A. niger)

By the same method as in Example 2, parts of the cytochrome b genes of strains of *A. niger* (*A. niger* IFM40606, 41398, 41399, 46897, 5367, 5368) were amplified by PCR and sequences thereof were determined. As the primer pair, the pair of DNA 2 and DNA 3 (for IFM41399) or the pair of DNA 2 and DNA 4 (for IFM40606, 41398, 46897, 5367, 5368) was used. In sequencing, DNA 2 was used as the forward primer and DNA 3 (for IFM41399) or DNA 4 (for IFM40606, 41398, 46897, 5367, 5368) was used as the reverse primer. The determined nucleotide sequences are shown in SEQ ID NOs. 36 to 41 in the SEQUENCE LISTING.

### Example 5 (Amplification Reaction of Cytochrome b Genes of A. niger)

By the same method as in Example 2, parts of the cytochrome b genes of strains of *A. nidulans* (*A. nidulans* IFM41094, 46999, 47004, 47006, 41395) were amplified by PCR and sequences thereof were determined. As the primer pair, the pair of DNA 2 and DNA 3 (for IFM47004, 47006) or the pair of DNA 2 and DNA 4 (for IFM41094, 46999, 41395) was used. In sequencing, DNA 2 was used as the forward primer and DNA 3 (for IFM47004, 47006) or DNA 4 (for IFM41094, 46999, 41395) was used as the reverse primer. The determined nucleotide sequences are shown in SEQ ID NOs. 42 to 46 in the SEQUENCE LISTING.

### Example 6 (Amplification Reaction of Cytochrome b Genes of A. terreus)

By the same method as in Example 2, parts of the cytochrome b genes of strains of *A. terreus* (*A. terreus* IFM40851, 40852, 41092, 5372, 40850) were amplified by PCR and sequences thereof were determined. As the primer pair, the pair of DNA 1 and DNA 3 was used. In sequencing, DNA 2 was used as the forward primer and DNA 3 was used as the reverse primer. The determined nucleotide sequences are shown in SEQ ID NOs. 47 to 51 in the SEQUENCE LISTING.

### Example 7 (Amplification Reaction of Cytochrome b Genes of C. kefyl)

By the same method as in Example 2, parts of the cytochrome b genes of strains of *C. kefyl* (*C. kefyl* IFM5733, 5800) were amplified by PCR and sequences thereof were determined. As the primer pair, the pair of DNA 2 and DNA 3 was used. In sequencing, DNA 2 was used as the forward primer and DNA 3 was used as the reverse primer. The determined nucleotide sequences are shown in SEQ ID NOs. 52 and 53 in the SEQUENCE LISTING.

### Example 8 (Amplification Reaction of Cytochrome b Genes of C. tropicalis)

By the same method as in Example 2, parts of the cytochrome b genes of strains of *C. tropicalis* (*C*. *tropicalis* IFM40018, 46816) were amplified by PCR and sequences thereof were determined. As the primer pair, the pair of DNA 1 and DNA 3 prepared in Example 1 was used. In sequencing, DNA 2 was used as the forward primer and DNA 3 was used as the reverse primer. The determined nucleotide sequences are shown in SEQ ID NOs. 54 and 55 in the SEQUENCE LISTING.

### Example 9 (Amplification Reaction of Cytochrome b Gene of trichophyton rubrum)

By the same method as in Example 2, a part of the cytochrome b gene of a strain of *Trichophyton rubrum* (*T. rubrum* IFM45593) was amplified by PCR and sequence thereof was determined. As the primer pair, the pair of DNA 1 and DNA 3 prepared in Example 1 was used. In sequencing, DNA 2 was used as the forward primer and DNA 3 was used as the reverse primer. The determined nucleotide sequence is shown in SEQ ID NO. 56 in the SEQUENCE LISTING.

### Example 10 (Identification of A. fumigatus by PCR)

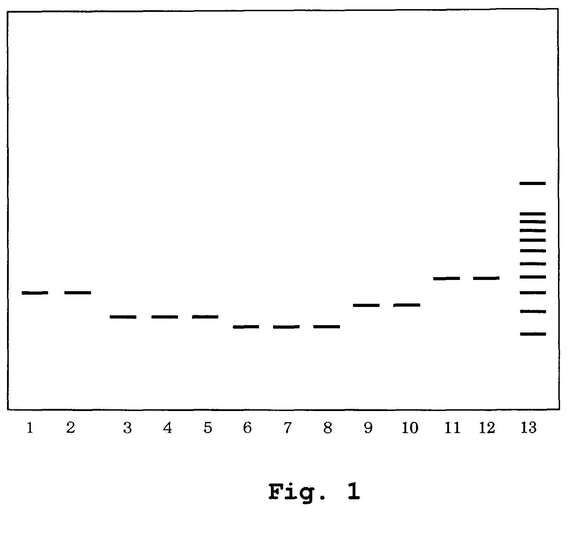

Parts of the cytochrome b genes of *A. fumigatus* were amplified by PCR as in Example 2 using the solutions of DNA 2 and DNA 10 (SEQ ID NO. 10) prepared in Example 1, and using TaKaRa PCR Amplification Kit (R011) commercially available from TAKARA SHUZO CO., LTD. and a DNA amplifier (MIR-D30) commercially available from SANYO. Nucleic acids of *A. fumigatus* were extracted by the phenol extraction method. Ten microliters of the solution containing each of the amplified products was electrophoresed on 1% agarose gel and the gel was stained with ethidium bromide, followed by detection of fluorescence during UV irradiation (Fig. 1, lanes 1 and 2). The electrophoresis was performed at a constant voltage of 75V for 45 minutes. In addition to the reaction mixture, molecular weight markers were also electrophoresed. Based on the relative mobility, the length of the detected nucleic acid fragment was calculated to be about 323 bp.

### Example 11 (Identification of A. flavus by PCR)

By the same method as in Example 10, using solution of DNA 2, and solutions of DNA 11 (SEQ ID NO. 11) and DNA 12 (SEQ ID NO. 12) prepared in Example 1, parts of the cytochrome b genes of *A. flavus* were amplified by PCR and the amplified products were detected by electrophoresis (Fig. 1, lanes 3, 4 and 5). The lengths of the detected nucleic acid fragments were calculated to be about 193 bp and or about 194 bp.

### Example 12 (Identification of A. niger by PCR)

By the same method as in Example 10, using solutions of DNA 2 and DNA 13 (SEQ ID NO. 13) prepared in Example 1, parts of the cytochrome b genes of *A. niger* were amplified by PCR and the amplified products were detected by electrophoresis (Fig. 1, lanes 6, 7 and 8). The length of the detected nucleic acid fragments was calculated to be about 148 bp.

### Example 13 (Identification of A. nidulans by PCR)

By the same method as in Example 10, using solutions of DNA 2 and DNA 14 (SEQ ID NO. 14) prepared in Example 1, parts of the cytochrome b genes of *A. nidulans* were amplified by PCR and the amplified products were detected by electrophoresis (Fig. 1, lanes 9 and 10). The length of the detected nucleic acid fragments was calculated to be about 244 bp.

### Example 14 (Identification of A. terreus by PCR)

By the same method as in Example 10, using solutions of DNA 2 and DNA 15 (SEQ ID NO. 15) prepared in Example 1, parts of the cytochrome b genes of *A. nidulans* were amplified by PCR and the amplified products were detected by electrophoresis (Fig. 1, lanes 11 and 12). The length of the detected nucleic acid fragments was calculated to be about 397 bp.

The lanes in Fig. 1 show the results of the following strains:
Lane 1: *A. fumigatus* IFM40819
Lane 2: *A. fumigatus* IFM40806
Lane 3: *A. flavus* IFM41933
Lane 4: *A. flavus* IFM5366
Lane 5: *A. flavus* IFM45910
Lane 6: *A. niger* IFM41398
Lane 7: *A. niger* IFM41399
Lane 8: *A. niger* IFM46897
Lane 9: *A. nidulans* IFM47004
Lane 10: *A. nidulans* IFM41094
Lane 11: *A. terreus* IFM40850
Lane 12: *A. terreus* IFM41092
Lane 13: size marker (100, 200, 300, 400, 500, 600, 700, 800, 900, 1000, 1500 bp)

### Example 15 (Amplification Reaction of Cytochrome b Gene of Cunninghamella bertholletiae)

By the method as in Example 2, a part of cytochrome b gene of *Cunninghamella bertholletiae* IFM 46114 was amplified and sequenced. As the primers, DNA57 and DNA58 were used. As primers for sequencing, DNA57 was used as a forward primer, and DNA58 was used as a reverse primer. The determined nucleotide sequence is shown in SEQ ID NO. 76 in the SEQUENCE LISTING.

### Example 16 (Amplification Reaction of Cytochrome b Gene of Mucor circinelloides)

By the method as in Example 2, a part of cytochrome b gene of *Mucor circinelloides* IFM 40507 was amplified and sequenced. As the primers, DNA57, and DNA60 and DNA61 were used. As primers for sequencing, DNA57 was used as a forward primer, and DNA60 and DNA61 were used as reverse primers. The determined nucleotide sequence is shown in SEQ ID NO. 77 in the SEQUENCE LISTING.

### Example 17

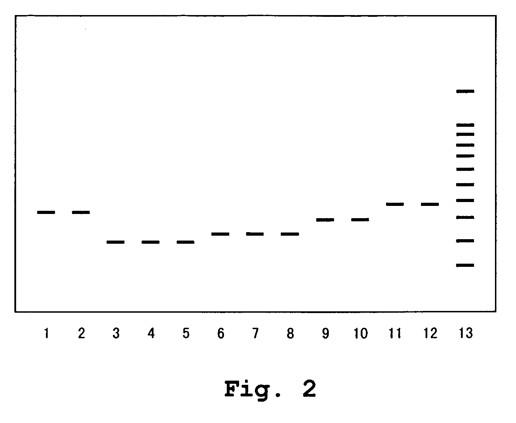

By the method as in Examples 10-14, identification of *Aspergillus* species was carried out. In each method, DNA57 was used as one of the primers. As other primers, DNA65 was used for identifying *Aspergillus fumigatus*, DNA11 and DNA12 were used for identifying *Aspergillus flavus*, DNA64 was used for identifying *Aspergillus niger*, DNA63 was used for identifying *Aspergillus nidulans*, and DNA62 was used for identifying *Aspergillus terreus*. The results of the electrophoresis are shown in Fig. 2. The strains of the respective lanes in Fig. 2 are the same as shown in Fig. 1.

### Example 18

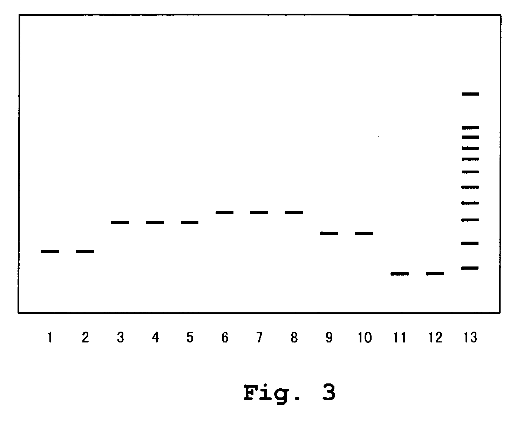

By the method as in Examples 10-14, identification of *Aspergillus* species was carried out. In each method, DNA3 was used as one of the primers. As other primers, DNA68 was used for identifying *Aspergillus fumigatus*, DNA66 and DNA67 were used for identifying *Aspergillus flavus*, DNA70 was used for identifying *Aspergillus niger*, DNA72 was used for identifying *Aspergillus nidulans*, and DNA74 was used for identifying *Aspergillus terreus*. The results of the electrophoresis are shown in Fig. 3. The strains of the respective lanes in Fig. 3 are the same as shown in Fig. 1.

### Example 19

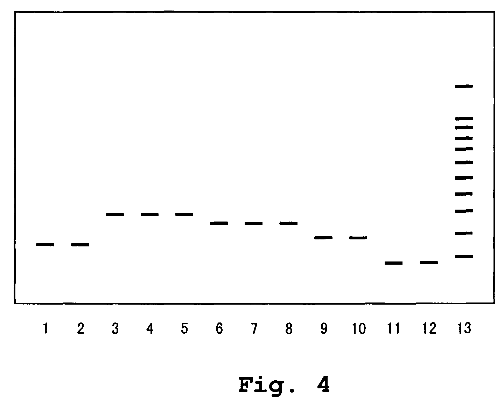

By the method as in Examples 10-14, identification of *Aspergillus* species was carried out. In each method, DNA58 was used as one of the primers. As other primers, DNA69 was used for identifying *Aspergillus fumigatus*, DNA66 and DNA67 were used for identifying *Aspergillus flavus*, DNA71 was used for identifying *Aspergillus niger*, DNA73 was used for identifying *Aspergillus nidulans*, and DNA75 was used for identifying *Aspergillus terreus*. The results of the electrophoresis are shown in Fig. 4. The strains of the respective lanes in Fig. 4 are the same as shown in Fig. 1.

### INDUSTRIAL AVAILABILITY

The present invention may be used for detection, grouping and identification of the pathogenic fungi, especially those belonging to the genus *Aspergillus*, quickly, simply, specifically and with high sensitivity. Therefore, the causative fungi may be rapidly identified for patients infected with pathogenic fungi, and appropriate therapy can be performed.

SEQUENCE LISTING

SEQ ID NO.: 1

SEQUENCE LENGTH: 20

SEQUENCE TYPE: nucleic acid

STRANDEDNESS: single

TOPOLOGY: linear

HYPOTHETICAL: No

SEQUENCE DESCRIPTION

TGAGGTGCTA CAGTTATTAC 20

SEQ ID NO.: 2

SEQUENCE LENGTH: 21

SEQUENCE TYPE: nucleic acid

STRANDEDNESS: single

TOPOLOGY: linear

HYPOTHETICAL: No

SEQUENCE DESCRIPTION

GAGGTGCTAC AGTTATTACT A 21

SEQ ID NO.: 3

SEQUENCE LENGTH: 20

SEQUENCE TYPE: nucleic acid

STRANDEDNESS: single

TOPOLOGY: linear

HYPOTHETICAL: No

SEQUENCE DESCRIPTION

GGTATAGMTC TTAAWATAGC 20

SEQ ID NO.: 4

SEQUENCE LENGTH: 20

SEQUENCE TYPE: nucleic acid

STRANDEDNESS: single

TOPOLOGY: linear

HYPOTHETICAL: No

SEQUENCE DESCRIPTION

AAAATAGCAT AGAAAGGTAA 20

SEQ ID NO.: 5

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus fumigatus

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTT 425

SEQ ID NO.: 6

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus

SEQUENCE DESCRIPTION

TGAGGTGCTA CAGTTATTAC TAACCTTATG AGTGCTATAC CTTGAATAGG TCAAGAYATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATYGCTATG 180

CACGATACTG TAGGATCTGG TAATCCTTTA GGTATATCTG GTAATTATGA TAGATTACCT 240

TTTGCTCCAT ATTTCATATT TAAAGATTTA GTWACTATCT TTATTTTCTT TATAGTATTA 300

TCTATATTTG TTTTCTTYAT GCCTAATGCT TTAGGAGATA GTGAAAATTA TGTTATGGCT 360

AATCCAATGC AAACTCCACC TGCTATYGTT CCAGAATGAT ACTTATTACC TTTCTATGCT 420

ATTTT 425

SEQ ID NO.: 7

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus niger

SEQUENCE DESCRIPTION

TGAGGTGCTA CAGTTATTAC TAACCTTATG AGTGCTATAC CATGAATAGG TCAAGATATA 60

GTTGAGTTCA TATGAGGWGG TTTCTCTGTA AATAATGCAA CATTAAACAG ATTCTTTGCA 120

TTACACTTCT TATTACCTTT TGTATTAGCT GCATTAGCTT TAATGCACTT AATAGCTATG 180

CACGATACAG TAGGATCAGG TAATCCATTA GGAATATCAG GTAATTACGA TAGATTACCT 240

TTTGCACCAT ATTTTATATT TAAAGATTTA GTAACTATCT TTATTTTCTT TATTGTATTA 300

TCAATATTTG TTTTCTTTAT GCCTAATGCA TTAGGAGATA GTGAAAATTA TGTTATGGCT 360

AAYCCAATGC AAACTCCACC TGCWATTGTA CCAGAGTGAT ATCTTTTACC TTTCTATGCT 420

ATTTT 425

SEQ ID NO.: 8

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus nidulans

SEQUENCE DESCRIPTION

TGAGGTGCTA CAGTTATTAC TAACCTAATG AGTGCTATAC CTTGAATAGG TCAAGATATT 60

GTTGAGTTTA TTTGAGGAGG TTTYTCTGTA AATAATGCAA CTTTAAACAG ATTCTTTGCA 120

TTACATTTCT TATTACCTTT TGTATTAGCT GCTTTAGCAT TAATGCATTT AATAGCTATG 180

CACGATACAG TAGGATCAGG KAATCCTTTA GGTATTTCAG CTAATTACGA TAGATTACCT 240

TTTGCTCCTT ATTTTATATT TAAAGATTTA ATAACTATAT TTATATTCTT TATTGTATTA 300

TCAATATTTG TTTTCTTTAT GCCTAATGCT TTAGGTGATA GTGAAAATTA TGTTATGGCT 360

AATCCTATGC AAACTCCACC TGCTATAGTT CCAGAATGAT ATCTTTTACC TTTCTATGCT 420

ATTTTA 425

SEQ ID NO.: 9

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus terreus

SEQUENCE DESCRIPTION

TGAGGTGCTA CAGTTATTAC TAACTTAATG AGTGCTATAC CTTGAATTGG TCAAGATATA 60

GTTGAATTTA TTTGAGGAGG TTTCTCTGTA AATAATGCAA CTTTAAATAG ATTCTTTGCA 120

TTACATTTCT TATTACCTTT TGTATTAGCT GCWTTAGTTA TTATGCACTT AATAGCAATG 180

CACGATACTG TAGGATCAGG TAATCCTTTA GGTATATCAG GTAACTACGA TAGATTACCT 240

TTCGCTCCAT ATTTCGTATT CAAAGATTTA GTAACTATCT TTATTTTCTT TATAGTATTA 300

TCTATATTTG TTTTCTTCAT GCCTAACGCA TTAGGAGACA GTGAAAATTA TGTTATGGCA 360

AACCCAATGC AAACACCACC TGCTATTGTA CCAGAATGAT ACTTATTACC ATTCTATGCT 420

ATTTTA、 425

SEQ ID NO.: 10

SEQUENCE LENGTH: 21

SEQUENCE TYPE: nucleic acid

STRANDEDNESS: single

TOPOLOGY: linear

HYPOTHETICAL: No

SEQUENCE DESCRIPTION

AGGCATGAAG AATACAAATA C 21

SEQ ID NO.: 11

SEQUENCE LENGTH: 22

SEQUENCE TYPE: nucleic acid

STRANDEDNESS: single

TOPOLOGY: linear
HYPOTHETICAL: No
SEQUENCE DESCRIPTION
TCCTACAGTA TCGTGCATAG CG 22

SEQ ID NO.: 12
SEQUENCE LENGTH: 21
SEQUENCE TYPE: nucleic acid
STRANDEDNESS: single
TOPOLOGY: linear
HYPOTHETICAL: No
SEQUENCE DESCRIPTION
CCTACAGTAT CGTGCATAGC A 21

SEQ ID NO.: 13
SEQUENCE LENGTH: 24
SEQUENCE TYPE: nucleic acid
STRANDEDNESS: single
TOPOLOGY: linear
HYPOTHETICAL: No
SEQUENCE DESCRIPTION
GCTAATACAA AAGGTAATAA GAAG 24

SEQ ID NO.: 14
SEQUENCE LENGTH: 25
SEQUENCE TYPE: nucleic acid
STRANDEDNESS: single

TOPOLOGY: linear

HYPOTHETICAL: No

SEQUENCE DESCRIPTION

GCAAAAGGTA ATCTATCGTA ATTAG 25

SEQ ID NO.: 15

SEQUENCE LENGTH: 24

SEQUENCE TYPE: nucleic acid

STRANDEDNESS: single

TOPOLOGY: linear

HYPOTHETICAL: No

SEQUENCE DESCRIPTION

CATTCTGGTA CAATAGCAGG TGGT 24

SEQ ID NO.: 16

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus fumigatus IFM40804

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360
AATCCAATGC AAACTCCACC TGCTATTGTT CCGGAATGAT ACTTATTACC TTTCTATGCT 420
ATTTTAAGAT CTATACC 437

SEQ ID NO.: 17
SEQUENCE LENGTH: 425
SEQUENCE TYPE: nucleic acid
STRANDEDNESS: both
TOPOLOGY: linear
HYPOTHETICAL: No
ORIGINAL SOURCE: Aspergillus fumigatus IFM40806
SEQUENCE DESCRIPTION
TGAGGTGCTA CAGTTATTAC TAATCTTATG AGTGCTATAC CTTGAATAGG TCAAGATATA 60
GTTGAATTTA TATGAGGTGG TTTCTCTGTA AATAATGCTA CATTAAATAG ATTCTTTGCA 120
TTACATTTCT TATTACCTTT TGTTTTAGCT GCATTAGTAA TAATGCATTT AATAGCAATG 180
CACGATACTG TAGGATCTGG TAATCCTTTA GGTATTTCTG GTAATTATGA TAGATTACCT 240
TTCGCTCCTT ACTTTGTATT TAAAGATTTA GTTACTGTAT TTATTTTCTT TATAGTATTA 300
TCTGTATTTG TATTCTTCAT GCCTAACGCA TTAGGTGATA GTGAAAATTA TGTTATGGCT 360
AATCCAATGC AAACTCCACC TGCTATTGTT CCGGAATGAT ACTTATTACC TTTCTATGCT 420
ATTTT 425

SEQ ID NO.: 18
SEQUENCE LENGTH: 425
SEQUENCE TYPE: nucleic acid
STRANDEDNESS: both
TOPOLOGY: linear
HYPOTHETICAL: No

ORIGINAL SOURCE: Aspergillus fumigatus IFM40807

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTT 425

SEQ ID NO.: 19

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus fumigatus IFM40819

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTTAAGAT CTATACC 437

SEQ ID NO.: 20

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus fumigatus IFM41206

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTT 425

SEQ ID NO.: 21

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus fumigatus IFM41392

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120
TTACATTTCT TATTACCTTT TGTTTTAGCT GCATTAGTAA TAATGCATTT AATAGCAATG 180
CACGATACTG TAGGATCTGG TAATCCTTTA GGTATTTCTG GTAATTATGA TAGATTACCT 240
TTCGCTCCTT ACTTTGTATT TAAAGATTTA GTTACTGTAT TTATTTTCTT TATAGTATTA 300
TCTGTATTTG TATTCTTCAT GCCTAACGCA TTAGGTGATA GTGAAAATTA TGTTATGGCT 360
AATCCAATGC AAACTCCACC TGCTATTGTT CCGGAATGAT ACTTATTACC TTTCTATGCT 420
ATTTT 425

SEQ ID NO.: 22
SEQUENCE LENGTH: 425
SEQUENCE TYPE: nucleic acid
STRANDEDNESS: both
TOPOLOGY: linear
HYPOTHETICAL: No
ORIGINAL SOURCE: Aspergillus fumigatus IFM45916
SEQUENCE DESCRIPTION
TGAGGTGCTA CAGTTATTAC TAATCTTATG AGTGCTATAC CTTGAATAGG TCAAGATATA 60
GTTGAATTTA TATGAGGTGG TTTCTCTGTA AATAATGCTA CATTAAATAG ATTCTTTGCA 120
TTACATTTCT TATTACCTTT TGTTTTAGCT GCATTAGTAA TAATGCATTT AATAGCAATG 180
CACGATACTG TAGGATCTGG TAATCCTTTA GGTATTTCTG GTAATTATGA TAGATTACCT 240
TTCGCTCCTT ACTTTGTATT TAAAGATTTA GTTACTGTAT TTATTTTCTT TATAGTATTA 300
TCTGTATTTG TATTCTTCAT GCCTAACGCA TTAGGTGATA GTGAAAATTA TGTTATGGCT 360
AATCCAATGC AAACTCCACC TGCTATTGTT CCGGAATGAT ACTTATTACC TTTCTATGCT 420
ATTTT 425

SEQ ID NO.: 23
SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus fumigatus IFM45917

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTT 425

SEQ ID NO.: 24

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus fumigatus IFM46980

SEQUENCE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTT 425

SEQ ID NO.: 25

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus fumigatus IFM5355

SEQUENGE DESCRIPTION

TGAGGTGCTA CAGTTATTAC TAATCTTATG AGTGCTATAC CTTGAATAGG TCAAGATATA 60

GTTGAATTTA TATGAGGTGG TTTCTCTGTA AATAATGCTA CATTAAATAG ATTCTTTGCA 120

TTACATTTCT TATTACCTTT TGTTTTAGCT GCATTAGTAA TAATGCATTT AATAGCAATG 180

CACGATACTG TAGGATCTGG TAATCCTTTA GGTATTTCTG GTAATTATGA TAGATTACCT 240

TTCGCTCCTT ACTTTGTATT TAAAGATTTA GTTACTGTAT TTATTTTCTT TATAGTATTA 300

TCTGTATTTG TATTCTTCAT GCCTAACGCA TTAGGTGATA GTGAAAATTA TGTTATGGCT 360

AATCCAATGC AAACTCCACC TGCTATTGTT CCGGAATGAT ACTTATTACC TTTCTATGCT 420

ATTTT 425

SEQ ID NO.: 26

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM40603

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTT 425

SEQ ID NO.: 27

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM40800

SEQUENCE DESCRIPTION

TGAGGTGCTA CAGTTATTAC TAACCTTATG AGTGCTATAC CTTGAATAGG TCAAGATATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTAACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATTGTT CCAGAATGAT ACTTATTACC TTTCTATGCT 420

ATTTT 425

SEQ ID NO.: 28

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM41087

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTT 425

SEQ ID NO.: 29

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus flavus IFM41933

SEQUENCE DESCRIPTION

TGAGGTGCTA CAGTTATTAC TAACCTTATG AGTGCTATAC CTTGAATAGG TCAAGATATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATTGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTCAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTTAAGAT CTATACC 437

SEQ ID NO.: 30

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM45909

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTT 425

SEQ ID NO.: 31

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus flavus IFM45910

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTTAAGAT CTATACC 437

SEQ ID NO.: 32

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM45911

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTT 425

SEQ ID NO.: 33

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM46870

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTT 425

SEQ ID NO.: 34

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus flavus IFM5364

SEQUENCE DESCRIPTION

TGAGGTGCTA CAGTTATTAC TAACCTTATG AGTGCTATAC CTTGAATAGG TCAAGACATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTTACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATCGTT CCAGAATGAT ACTTATTACC TTTCTATGCT 420

ATTTT 425

SEQ ID NO.: 35

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus flavus IFM5366

SEQUENCE DESCRIPTION

TGAGGTGCTA CAGTTATTAC TAACCTTATG AGTGCTATAC CTTGAATAGG TCAAGATATA 60

GTTGAATTTA TTTGAGGAGG TTTCTCTGTA AATAATGCAA CATTAAATAG ATTCTTTGCA 120

TTACATTTCT TATTACCTTT TGTATTAGCT GCATTAGCTT TAATGCATTT AATCGCTATG 180

CACGATACTG TAGGATCTGG TAATCCTTTA GGTATATCTG GTAATTATGA TAGATTACCT 240

TTTGCTCCAT ATTTCATATT TAAAGATTTA GTAACTATCT TTATTTTCTT TATAGTATTA 300

TCTATATTTG TTTTCTTTAT GCCTAATGCT TTAGGAGATA GTGAAAATTA TGTTATGGCT 360

AATCCAATGC AAACTCCACC TGCTATTGTT CCAGAATGAT ACTTATTACC TTTCTATGCT 420
ATTTTAAGAT CTATACC 437

SEQ ID NO.: 36
SEQUENCE LENGTH: 425
SEQUENCE TYPE: nucleic acid
STRANDEDNESS: both
TOPOLOGY: linear
HYPOTHETICAL: No
ORIGINAL SOURCE: Aspergillus niger IFM40606
SEQUENCE DESCRIPTION
TGAGGTGCTA CAGTTATTAC TAACCTTATG AGTGCTATAC CATGAATAGG TCAAGATATA 60
GTTGAGTTCA TATGAGGTGG TTTCTCTGTA AATAATGCAA CATTAAACAG ATTCTTTGCA 120
TTACACTTCT TATTACCTTT TGTATTAGCT GCATTAGCTT TAATGCACTT AATAGCTATG 180
CACGATACAG TAGGATCAGG TAATCCATTA GGAATATCAG GTAATTACGA TAGATTACCT 240
TTTGCACCAT ATTTTATATT TAAAGATTTA GTAACTATCT TTATTTTCTT TATTGTATTA 300
TCAATATTTG TTTTCTTTAT GCCTAATGCA TTAGGAGATA GTGAAAATTA TGTTATGGCT 360
AACCCAATGC AAACTCCACC TGCTATTGTA CCAGAGTGAT ATCTTTTACC TTTCTATGCT 420
ATTTT 425

SEQ ID NO.: 37
SEQUENCE LENGTH: 425
SEQUENCE TYPE: nucleic acid
STRANDEDNESS: both
TOPOLOGY: linear
HYPOTHETICAL: No
ORIGINAL SOURCE: Aspergillus niger IFM41398

SEQUENCE DESCRIPTION

TGAGGTGCTA CAGTTATTAC TAACCTTATG AGTGCTATAC CATGAATAGG TCAAGATATA 60

GTTGAGTTCA TATGAGGAGG TTTCTCTGTA AATAATGCAA CATTAAACAG ATTCTTTGCA 120

TTACACTTCT TATTACCTTT TGTATTAGCT GCATTAGCTT TAATGCACTT AATAGCTATG 180

CACGATACAG TAGGATCAGG TAATCCATTA GGAATATCAG GTAATTACGA TAGATTACCT 240

TTTGCACCAT ATTTTATATT TAAAGATTTA GTAACTATCT TTATTTTCTT TATTGTATTA 300

TCAATATTTG TTTTCTTTAT GCCTAATGCA TTAGGAGATA GTGAAAATTA TGTTATGGCT 360

AATCCAATGC AAACTCCACC TGCAATTGTA CCAGAGTGAT ATCTTTTACC TTTCTATGCT 420

ATTTT 425

SEQ ID NO.: 38

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus niger IFM41399

SEQUENCE DESCRIPTION

TGAGGTGCTA CAGTTATTAC TAACCTTATG AGTGCTATAC CATGAATAGG TCAAGATATA 60

GTTGAGTTCA TATGAGGAGG TTTCTCTGTA AATAATGCAA CATTAAACAG ATTCTTTGCA 120

TTACACTTCT TATTACCTTT TGTATTAGCT GCATTAGCTT TAATGCACTT AATAGCTATG 180

CACGATACAG TAGGATCAGG TAATCCATTA GGAATATCAG GTAATTACGA TAGATTACCT 240

TTTGCACCAT ATTTTATATT TAAAGATTTA GTAACTATCT TTATTTTCTT TATTGTATTA 300

TCAATATTTG TTTTCTTTAT GCCTAATGCA TTAGGAGATA GTGAAAATTA TGTTATGGCT 360

AATCCAATGC AAACTCCACC TGCAATTGTA CCAGAGTGAT ATCTTTTACC TTTCTATGCT 420

ATTTTAAGAT CTATACC 437

SEQ ID NO.: 39

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus niger IFM46897

SEQUENCE DESCRIPTION

TGAGGTGCTA CAGTTATTAC TAACCTTATG AGTGCTATAC CATGAATAGG TCAAGATATA 60

GTTGAGTTCA TATGAGGAGG TTTCTCTGTA AATAATGCAA CATTAAACAG ATTCTTTGCA 120

TTACACTTCT TATTACCTTT TGTATTAGCT GCATTAGCTT TAATGCACTT AATAGCTATG 180

CACGATACAG TAGGATCAGG TAATCCATTA GGAATATCAG GTAATTACGA TAGATTACCT 240

TTTGCAGCAT ATTTTATATT TAAAGATTTA GTAACTATCT TTATTTTCTT TATTGTATTA 300

TCAATATTTG TTTTCTTTAT GCCTAATGCA TTAGGAGATA GTGAAAATTA TGTTATGGCT 360

AATCCAATGC AAACTCCACC TGCAATTGTA CCAGAGTGAT ATCTTTTACC TTTCTATGCT 420

ATTTT 425

SEQ ID NO.: 40

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus niger IFM5367

SEQUENCE DESCRIPTION

TGAGGTGCTA CAGTTATTAC TAACCTTATG AGTGCTATAC CATGAATAGG TCAAGATATA 60

GTTGAGTTCA TATGAGGAGG TTTCTCTGTA AATAATGCAA CATTAAACAG ATTCTTTGCA 120

TTACACTTCT TATTACCTTT TGTATTAGCT GCATTAGCTT TAATGCACTT AATAGCTATG 180

CACGATACAG TAGGATCAGG TAATCCATTA GGAATATCAG GTAATTACGA TAGATTACCT 240

TTTGCACCAT ATTTTATATT TAAAGATTTA GTAACTATCT TTATTTTCTT TATTGTATTA 300

TCAATATTTG TTTTCTTTAT GCCTAATGCA TTAGGAGATA GTGAAAATTA TGTTATGGCT 360

AATCCAATGC AAACTCCACC TGCAATTGTA CCAGAGTGAT ATCTTTTACC TTTCTATGCT 420

ATTTT 425

SEQ ID NO.: 41

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus niger IFM5368

SEQUENCE DESCRIPTION

TGAGGTGCTA CAGTTATTAC TAACCTTATG AGTGCTATAC CATGAATAGG TCAAGATATA 60

GTTGAGTTCA TATGAGGAGG TTTCTCTGTA AATAATGCAA CATTAAACAG ATTCTTTGCA 120

TTACACTTCT TATTACCTTT TGTATTAGCT GCATTAGCTT TAATGCACTT AATAGCTATG 180

CACGATACAG TAGGATCAGG TAATCCATTA GGAATATCAG GTAATTACGA TAGATTACCT 240

TTTGCACCAT ATTTTATATT TAAAGATTTA GTAACTATCT TTATTTTCTT TATTGTATTA 300

TCAATATTTG TTTTCTTTAT GCCTAATGCA TTAGGAGATA GTGAAAATTA TGTTATGGCT 360

AATCCAATGC AAACTCCACC TGCAATTGTA CCAGAGTGAT ATCTTTTACC TTTCTATGCT 420

ATTTT 425

SEQ ID NO.: 42

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus nidulans IFM41094

SEQUENCE DESCRIPTION

TGAGGTGCTA CAGTTATTAC TAACCTAATG AGTGCTATAC CTTGAATAGG TCAAGATATT 60

GTTGAGTTTA TTTGAGGAGG TTTTTCTGTA AATAATGCAA CTTTAAACAG ATTCTTTGCA 120

TTACATTTCT TATTACCTTT TGTATTAGCT GCTTTAGCAT TAATGCATTT AATAGCTATG 180

CACGATACAG TAGGATCAGG TAATCCTTTA GGTATTTCAG CTAATTACGA TAGATTACCT 240

TTTGCTCCTT ATTTTATATT TAAAGATTTA ATAACTATAT TTATATTCTT TATTGTATTA 300

TCAATATTTG TTTTCTTTAT GCCTAATGCT TTAGGTGATA GTGAAAATTA TGTTATGGCT 360

AATCCTATGC AAACTCCACC TGCTATAGTT CCAGAATGAT ATCTTTTACC TTTCTATGCT 420

ATTTT 425

SEQ ID NO.: 43

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus nidulans IFM46999

SEQUENCE DESCRIPTION

TGAGGTGCTA CAGTTATTAC TAACCTAATG AGTGCTATAC CTTGAATAGG TCAAGATATT 60

GTTGAGTTTA TTTGAGGAGG TTTTTCTGTA AATAATGCAA CTTTAAACAG ATTCTTTGCA 120

TTACATTTCT TATTACCTTT TGTATTAGCT GCTTTAGCAT TAATGCATTT AATAGCTATG 180

CACGATACAG TAGGATCAGG TAATCCTTTA GGTATTTCAG CTAATTACGA TAGATTACCT 240

TTTGCTCCTT ATTTTATATT TAAAGATTTA ATAACTATAT TTATATTCTT TATTGTATTA 300

TCAATATTTG TTTTCTTTAT GCCTAATGCT TTAGGTGATA GTGAAAATTA TGTTATGGCT 360
AATCCTATGC AAACTCCACC TGCTATAGTT CCAGAATGAT ATCTTTTACC TTTCTATGCT 420
ATTTT 425

SEQ ID NO.: 44
SEQUENCE LENGTH: 437
SEQUENCE TYPE: nucleic acid
STRANDEDNESS: both
TOPOLOGY: linear
HYPOTHETICAL: No
ORIGINAL SOURCE: Aspergillus nidulans IFM47004
SEQUENCE DESCRIPTION
TGAGGTGCTA CAGTTATTAC TAACCTAATG AGTGCTATAC CTTGAATAGG TCAAGATATT 60
GTTGAGTTTA TTTGAGGAGG TTTCTCTGTA AATAATGCAA CTTTAAACAG ATTCTTTGCA 120
TTACATTTCT TATTACCTTT TGTATTAGCT GCTTTAGCAT TAATGCATTT AATAGCTATG 180
CACGATACAG TAGGATCAGG GAATCCTTTA GGTATTTCAG CTAATTACGA TAGATTACCT 240
TTTGCTCCTT ATTTTATATT TAAAGATTTA ATAACTATAT TTATATTCTT TATTGTATTA 300
TCAATATTTG TTTTCTTTAT GCCTAATGCT TTAGGTGATA GTGAAAATTA TGTTATGGCT 360
AATCCTATGC AAACTCCACC TGCTATAGTT CCAGAATGAT ATCTTTTACC TTTCTATGCT 420
ATTTTAAGAT CTATACC 437

SEQ ID NO.: 45
SEQUENCE LENGTH: 437
SEQUENCE TYPE: nucleic acid
STRANDEDNESS: both
TOPOLOGY: linear
HYPOTHETICAL: No

ORIGINAL SOURCE: Aspergillus nidulans IFM47006

SEQUENCE DESCRIPTION

TGAGGTGCTA CAGTTATTAC TAACCTAATG AGTGCTATAC CTTGAATAGG TCAAGATATT 60

GTTGAGTTTA TTTGAGGAGG TTTCTCTGTA AATAATGCAA CTTTAAACAG ATTCTTTGCA 120

TTACATTTCT TATTACCTTT TGTATTAGCT GCTTTAGCAT TAATGCATTT AATAGCTATG 180

CACGATACAG TAGGATCAGG GAATCCTTTA GGTATTTCAG CTAATTACGA TAGATTACCT 240

TTTGCTCCTT ATTTTATATT TAAAGATTTA ATAACTATAT TTATATTCTT TATTGTATTA 300

TCAATATTTG TTTTCTTTAT GCCTAATGCT TTAGGTGATA GTGAAAATTA TGTTATGGCT 360

AATCCTATGC AAACTCCACC TGCTATAGTT CCAGAATGAT ATCTTTTACC TTTCTATGCT 420

ATTTTAAGAT CTATACC 437

SEQ ID NO.: 46

SEQUENCE LENGTH: 425

SEQUENCE TYPE: nucleic acid

STRANDEDNESS: both

TOPOLOGY: linear

HYPOTHETICAL: No

ORIGINAL SOURCE: Aspergillus nidulans IFM41395

SEQUENCE DESCRIPTION

TGAGGTGCTA CAGTTATTAC TAACCTAATG AGTGCTATAC CTTGAATAGG TCAAGATATT 60

GTTGAGTTTA TTTGAGGAGG TTTTTCTGTA AATAATGCAA CTTTAAACAG ATTCTTTGCA 120

TTACATTTCT TATTACCTTT TGTATTAGCT GCTTTAGCAT TAATGCATTT AATAGCTATG 180

CACGATACAG TAGGATCAGG TAATCCTTTA GGTATTTCAG CTAATTACGA TAGATTACCT 240

TTTGCTCCTT ATTTTATATT TAAAGATTTA ATAACTATAT TTATATTCTT TATTGTATTA 300

TCAATATTTG TTTTCTTTAT GCCTAATGCT TTAGGTGATA GTGAAAATTA TGTTATGGCT 360

AATCCTATGC AAACTCCACC TGCTATAGTT CCAGAATGAT ATCTTTTACC TTTCTATGCT 420

ATTTT 425

SEQ ID NO.: 47

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus terreus IFM40851

SEQUENCE DESCRIPTION

TGAGGTGCTA CAGTTATTAC TAACTTAATG AGTGCTATAC CTTGAATTGG TCAAGATATA 60

GTTGAATTTA TTTGAGGAGG TTTCTCTGTA AATAATGCAA CTTTAAATAG ATTCTTTGCA 120

TTACATTTCT TATTACCTTT TGTATTAGCT GCATTAGTTA TTATGCACTT AATAGCAATG 180

CACGATACTG TAGGATCAGG TAATCCTTTA GGTATATCAG GTAACTACGA TAGATTACCT 240

TTCGCTCCAT ATTTCGTATT CAAAGATTTA GTAACTATCT TTATTTTCTT TATAGTATTA 300

TCTATATTTG TTTTCTTCAT GCCTAACGCA TTAGGAGACA GTGAAAATTA TGTTATGGCA 360

AACCCAATGC AAACACCACC TGCTATTGTA CCAGAATGAT ACTTATTACC ATTCTATGCT 420

ATTKTAAGAT CTMTACC 437

SEQ ID NO.: 48

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus terreus IFM40852

SEQUENCE DESCRIPTION

TGAGGTGCTA CAGTTATTAC TAACTTAATG AGTGCTATAC CTTGAATTGG TCAAGATATA 60

GTTGAATTTA TTTGAGGAGG TTTCTCTGTA AATAATGCAA CTTTAAATAG ATTCTTTGCA 120

TTACATTTCT TATTACCTTT TGTATTAGCT GCATTAGTTA TTATGCACTT AATAGCAATG 180

CACGATACTG TAGGATCAGG TAATCCTTTA GGTATATCAG GTAACTACGA TAGATTACCT 240

TTCGCTCCAT ATTTCGTATT CAAAGATTTA GTAACTATCT TTATTTTCTT TATAGTATTA 300

TCTATATTTG TTTTCTTCAT GCCTAACGCA TTAGGAGACA GTGAAAATTA TGTTATGGCA 360

AACCCAATGC AAACACCACC TGCTATTGTA CCAGAATGAT ACTTATTACC ATTCTATGCT 420

ATTKTAAGAT CTMTACC 437

SEQ ID NO.: 49

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus terreus IFM41092

SEQUENCE DESCRIPTION

TGAGGTGCTA CAGTTATTAC TAACTTAATG AGTGCTATAC CTTGAATTGG TCAAGATATA 60

GTTGAATTTA TTTGAGGAGG TTTCTCTGTA AATAATGCAA CTTTAAATAG ATTCTTTGCA 120

TTACATTTCT TATTACCTTT TGTATTAGCT GCTTTAGTTA TTATGCACTT AATAGCAATG 180

CACGATACTG TAGGATCAGG TAATCCTTTA GGTATATCAG GTAACTACGA TAGATTACCT 240

TTCGCTCCAT ATTTCGTATT CAAAGATTTA GTAACTATCT TTATTTTCTT TATAGTATTA 300

TCTATATTTG TTTTCTTCAT GCCTAACGCA TTAGGAGACA GTGAAAATTA TGTTATGGCA 360

AACCCAATGC AAACACCACC TGCTATTGTA CCAGAATGAT ACTTATTACC ATTCTATGCT 420

ATTKTAAGAT CTMTACC 437

SEQ ID NO.: 50

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus terreus IFM5372

SEQUENCE DESCRIPTION

TGAGGTGCTA CAGTTATTAC TAACTTAATG AGTGCTATAC CTTGAATTGG TCAAGATATA 60

GTTGAATTTA TTTGAGGAGG TTTCTCTGTA AATAATGCAA CTTTAAATAG ATTCTTTGCA 120

TTACATTTCT TATTACCTTT TGTATTAGCT GCATTAGTTA TTATGCACTT AATAGCAATG 180

CACGATACTG TAGGATCAGG TAATCCTTTA GGTATATCAG GTAACTACGA TAGATTACCT 240

TTCGCTCCAT ATTTCGTATT CAAAGATTTA GTAACTATCT TTATTTTCTT TATAGTATTA 300

TCTATATTTG TTTTCTTCAT GCCTAACGCA TTAGGAGACA GTGAAAATTA TGTTATGGCA 360

AACCCAATGC AAACACCACC TGCTATTGTA CCAGAATGAT ACTTATTACC ATTCTATGCT 420

ATTKTAAGAT CTMTACC 437

SEQ ID NO.: 51

SEQUENCE LENGTH: 437

SEQUENCE TYPE: nucleic acid

STRANDEDNESS: both

TOPOLOGY: linear

HYPOTHETICAL: No

ORIGINAL SOURCE: Aspergillus terreus IFM40850

SEQUENCE DESCRIPTION

TGAGGTGCTA CAGTTATTAC TAACTTAATG AGTGCTATAC CTTGAATTGG TCAAGATATA 60

GTTGAATTTA TTTGAGGAGG TTTCTCTGTA AATAATGCAA CTTTAAATAG ATTCTTTGCA 120

TTACATTTCT TATTACCTTT TGTATTAGCT GCATTAGTTA TTATGCACTT AATAGCAATG 180

CACGATACTG TAGGATCAGG TAATCCTTTA GGTATATCAG GTAACTACGA TAGATTACCT 240

TTCGCTCCAT ATTTCGTATT CAAAGATTTA GTAACTATCT TTATTTTCTT TATAGTATTA 300

TCTATATTTG TTTTCTTCAT GCCTAACGCA TTAGGAGACA GTGAAAATTA TGTTATGGCA 360

AACCCAATGC AAACACCACC TGCTATTGTA CCAGAATGAT ACTTATTACC ATTCTATGCT 420

ATTKTAAGAT CTMTACC 437

SEQ ID NO.: 52

SEQUENCE LENGTH: 434

SEQUENCE TYPE: nucleic acid

STRANDEDNESS: both

TOPOLOGY: linear

HYPOTHETICAL: No

ORIGINAL SOURCE: Candida kefyl IFM5773

SEQUENCE DESCRIPTION

TGAGGTGCTA CAGTTATTAC TAATTTATTC TCAGCTATTC CATTAATTGG ACAAGATATT 60

GTATTATGAT TATGAGGAGG TTTCTCAGTA TCTAACCCTA CAATTCAAAG ATTCTTTGCA 120

TTCCATTACT TAGTACCATT TATTATTGCT GCTTGTGTAA TTATGCATTT AATGGCTTTA 180

CATACACATG GTTCATCAAA TCCTTTAGGT GTAACAGGTA ATTTAGATAG ATTACCAATG 240

CATGGATACT TTATTTTTAA AGATTTAATT ACAGTATTTG TATTCTTAAT TTTCTTCTCA 300

TTATTTGTAT TCTTCTCACC TAATACATTA GGACATCCTG ATAACTATAT TCCAGGTAAT 360

CCATTAGTAA CACCAGCATC AATTGTACCT GAATGATACT TATTACCATT TTATGCTATW 420

TTAAGATCTA TACC 434

SEQ ID NO.: 53

SEQUENCE LENGTH: 434

SEQUENCE TYPE: nucleic acid

STRANDEDNESS: both

TOPOLOGY: linear

HYPOTHETICAL: No

ORIGINAL SOURCE: Candida kefyl IFM5800

SEQUENCE DESCRIPTION

TGAGGTGCTA CAGTTATTAC TAATTTATTC TCAGCTATTC CATTAATTGG ACAAGATATT 60

GTATTATGAT TATGAGGAGG TTTCTCAGTA TCTAACCCTA CAATTCAAAG ATTCTTTGCA 120

TTCCATTACT TAGTACCATT TATTATTGCT GCTTGTGTAA TTATGCATTT AATGGCTTTA 180

CATACACATG GTTCATCAAA TCCTTTAGGT GTAACAGGTA ATTTAGATAG ATTACCAATG 240

CATGGATACT TTATTTTTAA AGATTTAATT ACAGTATTTG TATTCTTAAT TTTCTTCTCA 300

TTATTTGTAT TCTTCTCACC TAATACATTA GGACATCCTG ATAACTATAT TCCAGGTAAT 360

CCATTAGTAA CACCAGCATC AATTGTACCT GAATGATACT TATTACCATT TTATGCTATT 420

TTAAGATCTA TACC 434

SEQ ID NO.: 54

SEQUENCE LENGTH: 434

SEQUENCE TYPE: nucleic acid

STRANDEDNESS: both

TOPOLOGY: linear

HYPOTHETICAL: No

ORIGINAL SOURCE: Candida tropicalis IFM40018

SEQUENCE DESCRIPTION

TGAGGTGCTA CAGTTATTAC TAACTTATTA TCAGCTATAC CATTTATAGG TAATGATATA 60

GTACCATTTA TATGAGGAGG TTTCTCTGTA TCAAATCCTA CAATACAAAG ATTTTTTGCA 120

TTACATTATT TATTACCATT TATTTTAGCA GCATTAGTAG TAATGCATTT TATAGCATTA 180

CATACTCATG GATCATCTAA TCCTGTAGGA ATATCAAGTA ATTTAGATAG AATACCAATG 240

CATAGTTACT TTATATTTAA AGATTTAATT ACTGTATTTG TATTTATATT AGTATTTAGT 300

TTATTTGTAT TCTTCTCACC TAATACTTTA GGACATCCTG ATAACTATAT ACCAGGTAAT 360

CCTATGGTTA CACCTGCATC TATAGTACCT GAATGATATT TATTACCATT CTATGCTATA 420

TTAAGATCTA TACC 434

SEQ ID NO.: 55

SEQUENCE LENGTH: 434

SEQUENCE TYPE: nucleic acid

STRANDEDNESS: both

TOPOLOGY: linear

HYPOTHETICAL: No

ORIGINAL SOURCE: Candida tropicalis IFM46816

SEQUENCE DESCRIPTION

TGAGGTGCTA CAGTTATTAC WAACTTATTA TCAGCTATAC CATTTATAGG TAATGATATA 60

GTACCATTTA TATGAGGAGG TTTCTCTGTA TCAAATCCTA CAATACAAAG ATTTTTTGCA 120

TTACATTATT TATTACCATT TATTTTAGCA GCATTAGTAG TAATGCATTT TATAGCATTA 180

CATACTCATG GATCATCTAA TCCTGTAGGA ATATCAAGTA ATTTAGATAG AATACCAATG 240

CATAGTTACT TTATATTTAA AGATTTAATT ACTGTATTTG TATTTATATT AGTATTTAGT 300

TTATTTGTAT TCTTCTCACC TAATACTTTA GGACATCCTG ATAACTATAT ACCAGGTAAT 360

CCTATGGTTA CACCTGCATC TATAGTACCT GAATGATATT TATTACCATT CTATGCTATW 420

TTAAGATCTA TACC 434

SEQ ID NO.: 56

SEQUENCE LENGTH: 437

SEQUENCE TYPE: nucleic acid

STRANDEDNESS: both

TOPOLOGY: linear

HYPOTHETICAL: No

ORIGINAL SOURCE: Trichophyton rubrum IFM45593

SEQUENCE DESCRIPTION

TGAGGTGCTA CAGTTATTAC TAATTTATTA AGTGCTATAC CTTGAATTGG TCAAGATTTA 60
GTAGAATTTA TTTGAGGAGG TTTTTCTGTA AATAATGCTA CATTAAATAG ATTTTTTTCT 120
TTACATTATT TATTACCTTT TATATTAGCT GCTTTAGTTT TAATGCATTT AATAGCATTA 180
CATGATACTG TTGGTTCTAG TAATCCTTTA GGTATTTCAG GTAATTATGA TAGATTACCT 240
TTTGCTCCTT ACTTTTTATT TAAAGATTTA GTTACTATTT TCTTATTTAT GCTTGGTTTA 300
TCTATATTTG TTTTCTTTAT GCCTAATGCT TTAGGTGACT GTGAAAATTA TGTAATGGCT 360
AATCCTATGC AAACACCAGC TGCTATTGTT CCTGAATGAT ATTTATTACC TTTTTATGCT 420
ATATTAAGAT CTATACC 437

SEQ ID NO.: 57
SEQUENCE LENGTH: 22
SEQUENCE TYPE: nucleic acid
STRANDEDNESS: single
TOPOLOGY: linear
HYPOTHETICAL: No
SEQUENCE DESCRIPTION
TGRGGWGCWA CWGTTATTAC TA 22

SEQ ID NO.: 58
SEQUENCE LENGTH: 22
SEQUENCE TYPE: nucleic acid
STRANDEDNESS: single
TOPOLOGY: linear
HYPOTHETICAL: No
SEQUENCE DESCRIPTION
GGWATAGMWS KTAAWAYAGC ATA 22

SEQ ID NO.: 59
SEQUENCE LENGTH: 20
SEQUENCE TYPE: nucleic acid
STRANDEDNESS: single
TOPOLOGY: linear
HYPOTHETICAL: No
SEQUENCE DESCRIPTION
GGTATAGMWS KTAAWAYAGC 20

SEQ ID NO.: 60
SEQUENCE LENGTH: 20
SEQUENCE TYPE: nucleic acid
STRANDEDNESS: single
TOPOLOGY: linear
HYPOTHETICAL: No
SEQUENCE DESCRIPTION
GGTATAGMWG TTAAWATAGC 20

SEQ ID NO.: 61
SEQUENCE LENGTH: 20
SEQUENCE TYPE: nucleic acid
STRANDEDNESS: single
TOPOLOGY: linear
HYPOTHETICAL: No
SEQUENCE DESCRIPTION
GGTATAGATC TTAAWATAGC 20

SEQ ID NO.: 62
SEQUENCE LENGTH: 21
SEQUENCE TYPE: nucleic acid
STRANDEDNESS: single
TOPOLOGY: linear
HYPOTHETICAL: No
SEQUENCE DESCRIPTION
GGTGGTGTTT GCATTGGGTT T 21

SEQ ID NO.: 63
SEQUENCE LENGTH: 25
SEQUENCE TYPE: nucleic acid
STRANDEDNESS: single
TOPOLOGY: linear
HYPOTHETICAL: No
SEQUENCE DESCRIPTION
CAATAAAGAA TATAAATATA GTTAT 25

SEQ ID NO.: 64
SEQUENCE LENGTH: 26
SEQUENCE TYPE: nucleic acid
STRANDEDNESS: single
TOPOLOGY: linear
HYPOTHETICAL: No
SEQUENCE DESCRIPTION
TATCGTAATT ACCTGATATT CCTAAT 26

SEQ ID NO.: 65

SEQUENCE LENGTH: 27

SEQUENCE TYPE: nucleic acid

STRANDEDNESS: single

TOPOLOGY: linear

HYPOTHETICAL: No

SEQUENCE DESCRIPTION

TGCGTTAGGC ATGAAGAATA CAAATAC 27

SEQ ID NO.: 66

SEQUENCE LENGTH: 24

SEQUENCE TYPE: nucleic acid

STRANDEDNESS: single

TOPOLOGY: linear

HYPOTHETICAL: No

SEQUENCE DESCRIPTION

GCATTAGCTT TAATGCATTT AATC 24

SEQ ID NO.: 67

SEQUENCE LENGTH: 24

SEQUENCE TYPE: nucleic acid

STRANDEDNESS: single

TOPOLOGY: linear

HYPOTHETICAL: No

SEQUENCE DESCRIPTION

GCATTAGCTT TAATGCATTT AATT 24

SEQ ID NO.: 68
SEQUENCE LENGTH: 30
SEQUENCE TYPE: nucleic acid
STRANDEDNESS: single
TOPOLOGY: linear
HYPOTHETICAL: No
SEQUENCE DESCRIPTION
CTGTATTTAT TTTCTTTATA GTATTATCTG 30

SEQ ID NO.: 69
SEQUENCE LENGTH: 21
SEQUENCE TYPE: nucleic acid
STRANDEDNESS: single
TOPOLOGY: linear
HYPOTHETICAL: No
SEQUENCE DESCRIPTION
ATAGTATTAT CTGTATTTGT A 21

SEQ ID NO.: 70
SEQUENCE LENGTH: 26
SEQUENCE TYPE: nucleic acid
STRANDEDNESS: single
TOPOLOGY: linear
HYPOTHETICAL: No
SEQUENCE DESCRIPTION
CATTAAACAG ATTCTTTGCA TTACAC 26

SEQ ID NO.: 71

SEQUENCE LENGTH: 21

SEQUENCE TYPE: nucleic acid

STRANDEDNESS: single

TOPOLOGY: linear

HYPOTHETICAL: No

SEQUENCE DESCRIPTION

GGATCAGGTA ATCCATTAGG A 21

SEQ ID NO.: 72

SEQUENCE LENGTH: 20

SEQUENCE TYPE: nucleic acid

STRANDEDNESS: single

TOPOLOGY: linear

HYPOTHETICAL: No

SEQUENCE DESCRIPTION

AATCCTTTAG GTATTTCAGC 20

SEQ ID NO.: 73

SEQUENCE LENGTH: 28

SEQUENCE TYPE: nucleic acid

STRANDEDNESS: single

TOPOLOGY: linear

HYPOTHETICAL: No

SEQUENCE DESCRIPTION

ATTTAAAGAT TTAATAACTA TATTTATA 28

SEQ ID NO.: 74

SEQUENCE LENGTH: 24

SEQUENCE TYPE: nucleic acid

STRANDEDNESS: single

TOPOLOGY: linear

HYPOTHETICAL: No

SEQUENCE DESCRIPTION

GTTATGGCAA ACCCAATGCA AACA 24

SEQ ID NO.: 75

SEQUENCE LENGTH: 21

SEQUENCE TYPE: nucleic acid

STRANDEDNESS: single

TOPOLOGY: linear

HYPOTHETICAL: No

SEQUENCE DESCRIPTION

ATGGCAAACC CAATGCAAAC A 21

SEQ ID NO.: 76

SEQUENCE LENGTH: 434

SEQUENCE TYPE: nucleic acid

STRANDEDNESS: both

TOPOLOGY: linear

HYPOTHETICAL: No

ORIGINAL SOURCE: Cunninghamella bertholletiae

SEQUENCE DESCRIPTION

TGGGGAGCAA CAGTTATTAC TAATTTATTA TCTGCTATTC CTTGGATTGG TAAAGATCTT 60

GTAGAATTTA TTTGGGGTGG TTTCAGTGTT GATAATGCTA CTTTAAATCG ATTCTTTAGT 120
CTACATTATT TATTACCATT CATTCTTGCT GCTTTAGCAG TTATGCATTT AATTGCAAAA 180
GAAGAAAATG GATCAAGTAA TCCATTAGGA ATTACAGCTA ATGCTGATAT GATTTATATG 240
CATCCATATT ATATCTTTAA AGATTTAGTA ACTATTTTCT TATTCTTCTT AGTATTAGGA 300
TTATTCTTAT TTTATGCTCC TAATAAATTA GGACATCCTG ATAATTATAT TCCAGCTAAT 360
CCAATGCAAA CTCCAGCTTC TATTGTTCCT GAGTGGTATC TATTACCATT TTATGCTATT 420
TTAASWKCTA TWCC 434

SEQ ID NO.: 77
SEQUENCE LENGTH: 434
SEQUENCE TYPE: nucleic acid
STRANDEDNESS: both
TOPOLOGY: linear
HYPOTHETICAL: No
ORIGINAL SOURCE: Mucor circinelloides
SEQUENCE DESCRIPTION
TGGGGAGCAA CAGTTATTAC TAACCTATTA TCGGCTATCC CTTGGATTGG TAAAGATTTA 60
GTAGAATTCA TCTGGGGAGG TTTCTCTGTA GATAATGCGA CATTAAACCG ATTCTTCAGT 120
CTTCACTACT TATTACCATT CATCTTAGCA GCTTTATCTT TAATGCATTT AATTGCTAAA 180
GAAGAAAATG GATCTAATAA TCCACTAGGA ATTACAGCAA ACACTGATAT GGTTTATCTT 240
CATCCTTATT ACGTATTTAA AGATTTAGTA ACAATTTTCT TATTCTTCTT AGTATTAGCT 300
TTATTCTTAT TCTATGCACC TAATAAATTA GGTCATCCTG ATAATTATAT TCCAGCTAAC 360
CCAATGCAAA CTCCTGCATC TATTGTACCA GAATGGTATC TATTACCTTT CTATGCTATT 420
TTAASWKCTA TWCC 434

## Claims

1. A primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 57 (provided that one or more thymine at optional site(s) may be substituted with uracil) or in the nucleotide sequence complementary thereto.

2. The primer according to claim 1, which consists essentially of a nucleic acid fragment of which nucleotide sequence is shown in SEQ ID NO. 57 (provided that one or more thymine at optional site(s) may be substituted with uracil) or the nucleotide sequence complementary thereto.

3. A primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 3 (provided that one or more thymine at optional site(s) may be substituted with uracil) or in the nucleotide sequence complementary thereto.

4. The primer according to claim 3, which consists essentially of a nucleic acid fragment of which nucleotide sequence is shown in SEQ ID NO. 3 (provided that one or more thymine at optional site(s) may be substituted with uracil) or the nucleotide sequence complementary thereto.

5. A primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 58 (provided that one or more thymine at optional site(s) may be substituted with uracil) or in the nucleotide sequence complementary thereto.

6. The primer according to claim 5, which consists essentially of a nucleic acid fragment of which nucleotide sequence is shown in SEQ ID NO. 58 (provided that one or more thymine at optional site(s) may be substituted with uracil) or the nucleotide sequence complementary thereto.

7. A primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 59 (provided that one or more thymine at optional site(s) may be substituted with uracil) or in the nucleotide sequence complementary thereto.

8. The primer according to claim 7, which consists essentially of a nucleic acid fragment of which nucleotide sequence is shown in SEQ ID NO. 59 (provided that one or more thymine at optional site(s) may be substituted with uracil) or the nucleotide sequence complementary thereto.

9. A primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 60 (provided that one or more thymine at optional site(s) may be substituted with uracil) or in the nucleotide sequence complementary thereto.

10. The primer according to claim 9, which consists essentially of a nucleic acid fragment of which nucleotide sequence is shown in SEQ ID NO. 60 (provided that one or more thymine at optional site(s) may be substituted with uracil) or the nucleotide sequence complementary thereto.

11. A primer for amplifying a fragment of mitochondrial cytochrome b gene of fungi, consisting essentially of a nucleic acid fragment of which nucleotide sequence is a sequence of not less than 10 consecutive nucleotides in the nucleotide sequence shown in SEQ ID NO. 61 (provided that one or more thymine at optional site(s) may be substituted with uracil) or in the nucleotide sequence complementary thereto.

12. The primer according to claim 11, which consists essentially of a nucleic acid fragment of which nucleotide sequence is shown in SEQ ID NO. 61 (provided that one or more thymine at optional site(s) may be substituted with uracil) or the nucleotide sequence complementary thereto.

13. The primer according to any one of claims 1 to 12, wherein said primer is a mixture of said nucleic acid fragments each of which has the nucleotide sequence shown by the respective SEQ ID NO. which is a general formula.

14. The primer according to any one of claims 1 to 13, wherein said fungi are those belonging to the genus *Aspergillus*.

15. The primer according to any one of claims 1 to 13, wherein said fungi are those belonging to the genus *Candida*.

16. The primer according to any one of claims 1 to 13, wherein said fungi are those belonging to the genus *Cunninghamella*.

17. The primer according to any one of claims 1 to 13, wherein said fungi are those belonging to the genus *Mucor*.

18. The primer according to claim 14, wherein said fungi belonging to the genus *Aspergillus* is selected from the group consisting of *Aspergillus fumigatus*, *Aspergillus flavus*, *Aspergillus niger*, *Aspergillus nidulans* and *Aspergillus terreus*.

19. A nucleic acid for detection of *Aspergillus fumigatu*s, consisting essentially of a nucleic acid fragment of which nucleotide sequence is 10 to 100 consecutive nucleotides in the nucleotide sequence shown in SEQ ID NO. 5 (provided that one or more thymine at optional site(s) may be substituted with uracil) or in the nucleotide sequence complementary thereto.

20. A nucleic acid for detection of *Aspergillus flavus*, consisting essentially of a nucleic acid fragment of which nucleotide sequence is a sequence of 10 to 100 consecutive nucleotides in the nucleotide sequence shown in SEQ ID NO. 6 (provided that one or more thymine at optional site(s) may be substituted with uracil) or in the nucleotide sequence complementary thereto.

21. A nucleic acid for detection of *Aspergillus niger*, consisting essentially of a nucleic acid fragment of which nucleotide sequence is a sequence of 10 to 100 consecutive nucleotides in the nucleotide sequence shown in SEQ ID NO. 7 (provided that one or more thymine at optional site(s) may be substituted with uracil) or in the nucleotide sequence complementary thereto.

22. A nucleic acid for detection of *Aspergillus nidulans*, consisting essentially of a nucleic acid fragment of which nucleotide sequence is a sequence of 10 to 100 consecutive nucleotides in the nucleotide sequence shown in SEQ ID NO. 8 (provided that one or more thymine at optional site(s) may be substituted with uracil) or in the nucleotide sequence complementary thereto.

23. A nucleic acid for detection of *Aspergillus terreus*, consisting essentially of a nucleic acid fragment of which nucleotide sequence is a sequence of 10 to 100 consecutive nucleotides in the nucleotide sequence shown in SEQ ID NO. 9 (provided that one or more thymine at optional site(s) may be substituted with uracil) or in the nucleotide sequence complementary thereto.

24. The nucleic acid for detection according to any one of claims 19 to 23, wherein said 10 to 100 consecutive nucleotides is 15 to 30 consecutive nucleotides.

25. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 24nt "T" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 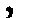 -end of said nucleic acid.

26. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 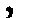 -end of said nucleic acid.

27. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 144nt "T" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 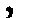 -end of said nucleic acid.

28. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 252nt "C" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 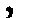 -end of said nucleic acid.

29. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 277nt "G" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 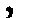 -end of said nucleic acid.

30. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 304nt "G" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 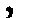 -end of said nucleic acid.

31. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 312nt "A" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 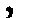 -end of said nucleic acid.

32. A nucleic acid for detection of *Aspergillus fumigatus*, consisting essentially of a nucleic acid fragment of which nucleotide sequence is a sequence of not less than 10 consecutive nucleotides in the nucleotide sequence shown in SEQ ID NO. 5, the 3'-end of said nucleic acid being the 393nt "G" in the nucleotide sequence shown in SEQ ID NO. 5, or consisting essentially of a nucleic acid fragment of which nucleotide sequence is a sequence of not less than 10 consecutive nucleotides in the nucleotide sequence complementary to the sequence shown in SEQ ID NO. 5, of which 3'-end is the base that pairs with said 3 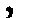 -end of said nucleic acid.

33. A nucleic acid for detection of *Aspergillus flavus*, consisting essentially of a nucleic acid fragment of which nucleotide sequence is a sequence of not less than 10 consecutive nucleotides in the nucleotide sequence shown in SEQ ID NO. 6, the 3'-end of said nucleic acid being the 174nt "Y" in the nucleotide sequence shown in SEQ ID NO. 6, or consisting essentially of a nucleic acid fragment of which nucleotide sequence is a sequence of not less than 10 consecutive nucleotides in the nucleotide sequence complementary to the sequence shown in SEQ ID NO. 6, of which 3'-end is the base that pairs with said 3 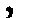 -end of said nucleic acid.

34. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 42nt "A" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 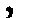 -end of said nucleic acid.

35. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 69nt "C" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 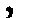 -end of said nucleic acid.

36. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 126nt "C" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 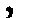 -end of said nucleic acid.

37. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 207nt "A" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 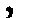 -end of said nucleic acid.

38. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 213nt "A" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 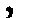 -end of said nucleic acid.

39. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 246nt "A" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 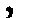 -end of said nucleic acid.

40. A nucleic acid for detection of *Aspergillus niger*, consisting essentially of a nucleic acid fragment of which nucleotide sequence is a sequence of not less than 10 consecutive nucleotides in the nucleotide sequence shown in SEQ ID NO. 7, the 3'-end of said nucleic acid being the 396nt "G" in the nucleotide sequence shown in SEQ ID NO. 7, or consisting essentially of a nucleic acid fragment of which nucleotide sequence is a sequence of not less than 10 consecutive nucleotides in the nucleotide sequence complementary to the sequence shown in SEQ ID NO. 7, of which 3'-end is the base that pairs with said 3 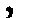 -end of said nucleic acid.

41. A nucleic acid for detection of *Aspergillus nidulans*, consisting essentially of a nucleic acid fragment of which nucleotide sequence is a sequence of not less than 10 consecutive nucleotides in the nucleotide sequence shown in SEQ ID NO. 8, the 3'-end of said nucleic acid being the 60nt "T" in the nucleotide sequence shown in SEQ ID NO. 8, or consisting essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with said 3 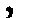 -end of said nucleic acid.

42. A nucleic acid for detection of *Aspergillus nidulans*, consisting essentially of a nucleic acid fragment of which nucleotide sequence is a sequence of not less than 10 consecutive nucleotides in the nucleotide sequence shown in SEQ ID NO. 8, the 3'-end of said nucleic acid being the 221nt "C" in the nucleotide sequence shown in SEQ ID NO. 8, or consisting essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with said 3 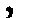 -end of said nucleic acid.

43. A nucleic acid for detection of *Aspergillus nidulans*, consisting essentially of a nucleic acid fragment of which nucleotide sequence is a sequence of not less than 10 consecutive nucleotides in the nucleotide sequence shown in SEQ ID NO. 8, the 3'-end of said nucleic acid being the 271nt "A" in the nucleotide sequence shown in SEQ ID NO. 8, or consisting essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with said 3 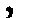 -end of said nucleic acid.

44. A nucleic acid for detection of *Aspergillus nidulans*, consisting essentially of a nucleic acid fragment of which nucleotide sequence is a sequence of not less than 10 consecutive nucleotides in the nucleotide sequence shown in SEQ ID NO. 8, the 3'-end of said nucleic acid being the 285nt "A" in the nucleotide sequence shown in SEQ ID NO. 8, or consisting essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with said 3 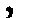 -end of said nucleic acid.

45. A nucleic acid for detection of *Aspergillus nidulans*, consisting essentially of a nucleic acid fragment of which nucleotide sequence is a sequence of not less than 10 consecutive nucleotides in the nucleotide sequence shown in SEQ ID NO. 8, the 3'-end of said nucleic acid being the 366nt "T" in the nucleotide sequence shown in SEQ ID NO. 8, or consisting essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with said 3 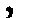 -end of said nucleic acid.

46. A nucleic acid for detection of *Aspergillus nidulans*, consisting essentially of a nucleic acid fragment of which nucleotide sequence is a sequence of not less than 10 consecutive nucleotides in the nucleotide sequence shown in SEQ ID NO. 8, the 3'-end of said nucleic acid being the 387nt "A" in the nucleotide sequence shown in SEQ ID NO. 8, or consisting essentially of a nucleic acid fragment of which nucleotide sequence is a sequence of not less than 10 consecutive nucleotides in the nucleotide sequence complementary to the sequence shown in SEQ ID NO. 8, of which 3'-end is the base that pairs with said 3 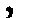 -end of said nucleic acid.

47. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 25nt "T" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 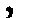 -end of said nucleic acid.

48. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 48nt "T" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 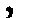 -end of said nucleic acid.

49. A nucleic acid for detection of *Aspergillus terreus*, consisting essentially of a nucleic acid fragment of which nucleotide sequence is a sequence of not less than 10 consecutive nucleotides in the nucleotide sequence shown in SEC ID NO. 9, the 3'-end of said nucleic acid being the 162nt "T" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 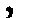 -end of said nucleic acid.

50. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 225nt "C" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 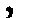 -end of said nucleic acid.

51. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 261nt "C" in the nucleotide sequence shown in SEC ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 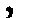 -end of said nucleic acid.

52. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 339nt "C" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 10, of which 3'-end is the base that pairs with said 3 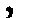 -end of said nucleic acid.

53. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 360nt "A" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 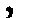 -end of said nucleic acid.

54. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 375nt "A" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 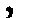 -end of said nucleic acid.

55. A nucleic acid for detection of *Aspergillus terreus*, consisting essentially of a nucleic acid fragment of which nucleotide sequence is a sequence of not less than 10 consecutive nucleotides in the nucleotide sequence shown in SEQ ID NO. 9, the 3'-end of said nucleic acid being the 411nt "A" in the nucleotide sequence shown in SEQ ID NO. 9, or consisting essentially of a nucleic acid fragment of which nucleotide sequence is a sequence of not less than 10 consecutive nucleotides in the nucleotide sequence complementary to the sequence shown in SEQ ID NO. 9, of which 3'-end is the base that pairs with said 3 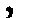 -end of said nucleic acid.

56. The nucleic acid according to any one of claims 25 to 55, wherein the number of nucleotides therein is 10 to 100.

57. The nucleic acid according to claim 56, wherein the number of nucleotides therein is 15 to 30.

58. The nucleic acid according to any one of claims 25 to 57, wherein said nucleic acid is a primer for amplification of a nucleic acid.

59. The nucleic acid according to any one of claims 25 to 57, wherein said nucleic acid is a labeled probe.

60. The nucleic acid for detection of *Aspergillus fumigatus* according to claim 30, wherein said nucleic acid consists essentially of a nucleic acid fragment of which nucleotide sequence is the sequence of shown in SEQ ID NO. 10 (provided that one or more thymine at optional site(s) may be substituted with uracil), or consists essentially of a nucleic acid fragment complementary to said nucleic acid.

61. The nucleic acid for detection of *Aspergillus flavus* according to claim 33, wherein said nucleic acid consists essentially of a nucleic acid fragment of which nucleotide sequence is the sequence of shown in SEQ ID NO. 11 (provided that one or more thymine at optional site(s) may be substituted with uracil), or consists essentially of a nucleic acid fragment complementary to said nucleic acid.

62. The nucleic acid for detection of *Aspergillus flavus* according to claim 33, wherein said nucleic acid consists essentially of a nucleic acid fragment of which nucleotide sequence is the sequence of shown in SEQ ID NO. 12 (provided that one or more thymine at optional site(s) may be substituted with uracil), or consists essentially of a nucleic acid fragment complementary to said nucleic acid.

63. The nucleic acid for detection of *Aspergillus niger* according to claim 36, wherein said nucleic acid consists essentially of a nucleic acid fragment of which nucleotide sequence is the sequence of shown in SEQ ID NO. 13 (provided that one or more thymine at optional site(s) may be substituted with uracil), or consists essentially of a nucleic acid fragment complementary to said nucleic acid.

64. The nucleic acid for detection of *Aspergillus nidulans* according to claim 42, wherein said nucleic acid consists essentially of a nucleic acid fragment of which nucleotide sequence is the sequence of shown in SEQ ID NO. 14 (provided that one or more thymine at optional site(s) may be substituted with uracil), or consists essentially of a nucleic acid fragment complementary to said nucleic acid.

65. The nucleic acid for detection of *Aspergillus terreus* according to claim 54, wherein said nucleic acid consists essentially of a nucleic acid fragment of which nucleotide sequence is the sequence of shown in SEQ ID NO. 15 (provided that one or more thymine at optional site(s) may be substituted with uracil), or consists essentially of a nucleic acid fragment complementary to said nucleic acid.

66. The nucleic acid for detection of *Aspergillus terreus* according to claim 53, wherein said nucleic acid consists essentially of a nucleic acid fragment of which nucleotide sequence is the sequence of shown in SEQ ID NO. 62 (provided that one or more thymine at optional site(s) may be substituted with uracil), or consists essentially of a nucleic acid fragment complementary to said nucleic acid.

67. The nucleic acid for detection of *Aspergillus nidulans* according to claim 43, wherein said nucleic acid consists essentially of a nucleic acid fragment of which nucleotide sequence is the sequence of shown in SEQ ID NO. 63 (provided that one or more thymine at optional site(s) may be substituted with uracil), or consists essentially of a nucleic acid fragment complementary to said nucleic acid.

68. The nucleic acid for detection of *Aspergillus niger* according to claim 37, wherein said nucleic acid consists essentially of a nucleic acid fragment of which nucleotide sequence is the sequence of shown in SEQ ID NO. 64 (provided that one or more thymine at optional site(s) may be substituted with uracil), or consists essentially of a nucleic acid fragment complementary to said nucleic acid.

69. The nucleic acid for detection of *Aspergillus fumigatus* according to claim 30, wherein said nucleic acid consists essentially of a nucleic acid fragment of which nucleotide sequence is the sequence of shown in SEQ ID NO. 65 (provided that one or more thymine at optional site(s) may be substituted with uracil), or consists essentially of a nucleic acid fragment complementary to said nucleic acid.

70. The nucleic acid for detection of *Aspergillus flavus* according to claim 37, wherein said nucleic acid consists essentially of a nucleic acid fragment of which nucleotide sequence is the sequence of shown in SEQ ID NO. 66 (provided that one or more thymine at optional site(s) may be substituted with uracil), or consists essentially of a nucleic acid fragment complementary to said nucleic acid.

71. The nucleic acid for detection of *Aspergillus flavus* according to claim 37, wherein said nucleic acid consists essentially of a nucleic acid fragment of which nucleotide sequence is the sequence of shown in SEQ ID NO. 67 (provided that one or more thymine at optional site(s) may be substituted with uracil), or consists essentially of a nucleic acid fragment complementary to said nucleic acid.

72. The nucleic acid for detection of *Aspergillus* *fumigatus* according to claim 30, wherein said nucleic acid consists essentially of a nucleic acid fragment of which nucleotide sequence is the sequence of shown in SEQ ID NO. 68 (provided that one or more thymine at optional site(s) may be substituted with uracil), or consists essentially of a nucleic acid fragment complementary to said nucleic acid.

73. The nucleic acid for detection of *Aspergillus fumigatus* according to claim 31, wherein said nucleic acid consists essentially of a nucleic acid fragment of which nucleotide sequence is the sequence of shown in SEQ ID NO. 69 (provided that one or more thymine at optional site(s) may be substituted with uracil), or consists essentially of a nucleic acid fragment complementary to said nucleic acid.

74. The nucleic acid for detection of *Aspergillus niger* according to claim 36, wherein said nucleic acid consists essentially of a nucleic acid fragment of which nucleotide sequence is the sequence of shown in SEQ ID NO. 70 (provided that one or more thymine at optional site(s) may be substituted with uracil), or consists essentially of a nucleic acid fragment complementary to said nucleic acid.

75. The nucleic acid for detection of *Aspergillus niger* according to claim 38, wherein said nucleic acid consists essentially of a nucleic acid fragment of which nucleotide sequence is the sequence of shown in SEQ ID NO. 71 (provided that one or more thymine at optional site(s) may be substituted with uracil), or consists essentially of a nucleic acid fragment complementary to said nucleic acid.

76. The nucleic acid for detection of *Aspergillus nidulans* according to claim 42, wherein said nucleic acid consists essentially of a nucleic acid fragment of which nucleotide sequence is the sequence of shown in SEQ ID NO. 72 (provided that one or more thymine at optional site(s) may be substituted with uracil), or consists essentially of a nucleic acid fragment complementary to said nucleic acid.

77. The nucleic acid for detection of *Aspergillus nidulans* according to claim 44, wherein said nucleic acid consists essentially of a nucleic acid fragment of which nucleotide sequence is the sequence of shown in SEQ ID NO. 73 (provided that one or more thymine at optional site(s) may be substituted with uracil), or consists essentially of a nucleic acid fragment complementary to said nucleic acid.

78. The nucleic acid for detection of *Aspergillus terreus* according to claim 54, wherein said nucleic acid consists essentially of a nucleic acid fragment of which nucleotide sequence is the sequence of shown in SEQ ID NO. 74 (provided that one or more thymine at optional site(s) may be substituted with uracil), or consists essentially of a nucleic acid fragment complementary to said nucleic acid.

79. The nucleic acid for detection of *Aspergillus terreus* according to claim 54, wherein said nucleic acid consists essentially of a nucleic acid fragment of which nucleotide sequence is the sequence of shown in SEQ ID NO. 75 (provided that one or more thymine at optional site(s) may be substituted with uracil), or consists essentially of a nucleic acid fragment complementary to said nucleic acid.

80. The primer according to claim 1 or 2, which is a forward primer.

81. The nucleic acid according to any one of claims 70 to 79, which is a forward primer.

82. The primer according to any one of claims 3 to 11, which is a reverse primer.

83. The nucleic acid according to any one of claims 60 to 69, which is a reverse primer.

84. A method for detecting and/or identifying fungi belonging to the genus *Aspergillus* comprising amplifying a fragment of mitochondrial cytochrome b gene of fungi belonging to the genus *Aspergillus* by a gene amplification method using at least one forward primer selected from the group consisting of nucleic acid fragments of which nucleotide sequence is a sequence of not less than 10 consecutive nucleotides in the nucleotide sequence shown in SEQ ID NO. 1 or 2 (provided that one or more thymine at optional site(s) may be substituted with uracil) and the forward primer according to claim 80, and at least one reverse primer according to claim 83.

85. A method for detecting and/or identifying fungi belonging to the genus *Aspergillus* comprising amplifying a fragment of mitochondrial cytochrome b gene of fungi belonging to the genus *Aspergillus* by a gene amplification method using at least one forward primer according to claim 81, and at least one reverse primer selected from the group consisting of the reverse primer according to claim 82 and nucleic acid fragments of which nucleotide sequence is a sequence of not less than 10 consecutive nucleotides in the nucleotide sequence shown in SEQ ID NO. 4 (provided that one or more thymine at optional site (s) may be substituted with uracil).

86. A method for detecting and/or identifying fungi belonging to the genus *Aspergillus* comprising amplifying a fragment of mitochondrial cytochrome b gene of fungi belonging to the genus *Aspergillus* by a gene amplification method using at least one forward primer selected from the group consisting of nucleic acid fragments of which nucleotide sequence is a sequence of not less than 10 consecutive nucleotides in the nucleotide sequence shown in SEQ ID NO. 1 or 2 (provided that one or more thymine at optional site(s) may be substituted with uracil) and the forward primer according to claim 80, and at least one reverse primer selected from the group consisting of the reverse primer according to claim 82 and nucleic acid fragments of which nucleotide sequence is a sequence of not less than 10 consecutive nucleotides in the nucleotide sequence shown in SEQ ID NO. 4 (provided that one or more thymine at optional site(s) may be substituted with uracil).

87. A DNA fragment having a nucleotide sequence shown in any one of SEQ ID NOs. 5-9.
